# EUROPÄISCHE PATENTSCHRIFT

(11) **EP 1 960 544 B1**
(45) Veröffentlichungstag und Bekanntmachung des Hinweises auf die Patenterteilung: **20.02.2013**
(21) Anmeldenummer: 06829534.4
(22) Anmeldetag: 12.12.2006
(51) Int. Cl.: C12Q 1/68

(54) **MOLEKULARE MARKER FÜR EINE TUMORDIAGNOSE UND -THERAPIE**
MOLECULAR MARKERS FOR THE DIAGNOSIS AND TREATMENT OF TUMORS
M,ARQUEUR MOLECULAIRE POUR LE DIAGNOSTIC ET LA THERAPIE DES TUMEURS

(30) Priorität: 12.12.2005 DE 102005059242
(43) Veröffentlichungstag der Anmeldung: 27.08.2008
(73) Patentinhaber: Steiner, Eric, 65193 Wiesbaden (DE); Hengstler, Jan, 44225 Dortmund (DE); Sagemüller, Jens, 41516 Grevenbroich-Kapellen (DE)
(72) Erfinder: Steiner, Eric, 65193 Wiesbaden (DE); Hengstler, Jan, 44225 Dortmund (DE); Sagemüller, Jens, 41516 Grevenbroich-Kapellen (DE)
(74) Vertreter: Schnappauf, Georg
(86) Internationale Anmeldenummer: PCT/EP2006/011953
(87) Internationale Veröffentlichungsnummer: WO 2007/068449

(56) Entgegenhaltungen:
- WO-A-00/12758
- WO-A-99/45147
- WO-A-2004/081199
- WO-A-2005/061725
- WO-A2-01/96388
- WO-A2-02/081516
- DATABASE EMBL [Online] 17. Mai 2004 (2004-05-17), "17000600332003 GRN_PRENEU Homo sapiens cDNA 5', mRNA sequence." XP002430975 gefunden im EBI accession no. EMBL:CN260473 Database accession no. CN260473
- DATABASE EMBL [Online] 21. Oktober 2005 (2005-10-21), "Homo sapiens cDNA clone TESTI2009368, 5' end, mRNA sequence." XP002430976 gefunden im EBI accession no. EMBL:DB027004 Database accession no. DB027004
- DATABASE Geneseq [Online] 20. Mai 2004 (2004-05-20), "Human ovarian cancer DNA marker #21000." XP002443973 gefunden im EBI accession no. GSN:ADL62788 Database accession no. ADL62788 -& WO 01/70979 A2 (MILLENNIUM PREDICTIVE MEDICINE [US] MILLENNIUM PHARM INC [US]) 27. September 2001 (2001-09-27)
- DATABASE Geneseq [Online] 18. November 2004 (2004-11-18), "Human Narc16b (64549) cDNA." XP002443974 gefunden im EBI accession no. GSN:ADR40144 Database accession no. ADR40144 & WO 2004/072242 A (MILLENNIUM PHARM INC [US]; KELLY LOUISE M [US]; CARROLL JOSEPH M [US];) 26. August 2004 (2004-08-26)
- DATABASE Geneseq [Online] 18. November 2004 (2004-11-18), "Human Narc16b (64549) protein." XP002443975 gefunden im EBI accession no. GSP:ADR40145 Database accession no. ADR40145 & WO 2004/072242 A (MILLENNIUM PHARM INC [US]; KELLY LOUISE M [US]; CARROLL JOSEPH M [US];) 26. August 2004 (2004-08-26)
- DATABASE Geneseq [Online] 1. Juli 2004 (2004-07-01), "Antipsoriatic cDNA sequence #801." XP002443976 gefunden im EBI accession no. GSN:ADN05171 Database accession no. ADN05171 & WO 2004/028479 A (GENENTECH INC [US]; BODARY SARAH [US]; CLARK HILARY [US]; JACKMAN JANE) 8. April 2004 (2004-04-08)
- DATABASE Geneseq [Online] 1. Juli 2004 (2004-07-01), "Antipsoriatic protein sequence #765." XP002443977 gefunden im EBI accession no. GSP:ADN05172 Database accession no. ADN05172 & WO 2004/028479 A (GENENTECH INC [US]; BODARY SARAH [US]; CLARK HILARY [US]; JACKMAN JANE) 8. April 2004 (2004-04-08)
- DATABASE UniProt [Online] 10. Oktober 2002 (2002-10-10), "Putative glycerophosphodiester phosphodiesterase 5 (EC 3.1.-.-)." XP002443978 gefunden im EBI accession no. UNIPROT:Q9NPB8 Database accession no. Q9NPB8

## Beschreibung

Trotz interdisziplinärer Ansätze und Ausreizung klassischer Therapiemodalitäten gehören Krebserkrankungen weiterhin zu den führenden Todesursachen.

Eine Metastasierung ist einer der kritischsten Faktoren, die für das Versagen einer Krebstherapie verantwortlich sind. Obwohl eine Darstellung der Proteinexpression, Gen-Array-Analyse und Bestimmung kritischer Faktoren in Tumorgewebe die prognostische Klassifizierung von Tumoren verbessert haben, ist es nach wie vor schwierig, das Risiko einer Metastasierung durch Untersuchung des resezierten Primärtumors vorherzusagen (Jacquemier J et al., Cancer Res. 65:767-779, 2005; Garber K, Science 303:1754-5, 2004; Hengstler JG et al., Cancer Res. 59, 3206-3214, 1999; Hengstler JG et al., Int. J. Cancer 84, 388-395, 1999; Hengstler JG et al., Int. J. Cancer, 95, 121-127, 2001).

Ein typisches Beispiel ist Krebs des Endometriums, die häufigste Malignität des weiblichen Genitaltrakts. Nach einer vollständigen Tumorresektion hängt ein Überleben gewöhnlich von einem Auftreten von Metastasen ab. Stellen eines Wiederaufiretens bei Krebs des Endometriums sind para-aortale Lymphknoten, Knochen, Lunge, Becken, Leber und Vagina (Steiner E et al., Int J Gynecol Cancer 13:197-203, 2003). Gegenwärtig ist es schwierig vorherzusagen, ob ein Primärtumor des Endometriums metastasiert hat oder nicht.

Die Faktoren, die eine Einstellung des metastatischen Phänotyps regulieren, sind größtenteils undefiniert. Es ist bekannt, dass manche histopathologischen Parameter wie das Tumorstadium und der histologische Grad mit einem tumorfreien Überleben assoziiert sind (Steiner E et al., Int J Gynecol Cancer 13:197-203, 2003). Jedoch ist es bisher unmöglich, das Risiko für eine Metastasierung durch Quantifizierung kritischer Faktoren in Tumorgewebe vorherzusagen.

Die WO 00/12758 beschreibt ein Verfahren für einen Nachweis, eine Diagnose, Überwachung, Klassifikation, Prognose, Darstellung und Behandlung von bestimmten Krebstypen, einschließlich gynäkologischer Krebstypen wie Brustkrebs, Ovarialkrebs, Uteruskrebs und Krebs des Endometriums und Lungenkrebs.

Die WO 01/70979 beschreibt Zusammensetzungen, Kits und Verfahren für einen Nachweis, eine Charakterisierung, Vermeidung und Behandlung von Ovarialkrebs des Menschen.

Die WO 01/96388 beschreibt Zusammensetzungen und Verfahren für eine Therapie und Diagnose von Kolonkrebs.

Es war die Aufgabe der vorliegenden Erfindung, Zielstrukturen für eine Diagnose, Prognose und Therapie von Krebserkrankungen bereitzustellen. Insbesondere war die Aufgabe der vorliegenden Erfindung die Identifizierung molekularer Marker, die eine Differentialdiagnose zwischen metastasierenden und nicht-metastasierenden Tumoren, insbesondere endometrialen Tumoren, ermöglichen.

Diese Aufgabe wird erfindungs gemäß durch den Gegenstand der Patentansprüche gelöst.

In diese Patentanmeldung werden genetische Marker identifiziert, deren Expression mit einem metastatischen Verhalten von Krebs des Endometriums korreliert. Solche genetischen Marker betreffen Nukleinsäuren, die ausgewählt sind aus der Gruppe bestehend aus (a) einer Nukleinsäure, die eine Nukleinsäuresequenz umfasst, die aus der Gruppe bestehend aus SEQ ID NO: 1-7, und einem Teil von mindestens 30 aufeinanderfolgenden Nukleotiden davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist.

Die vorliegende Erfindung betrifft allgemein die Diagnose, Prognose, und Überwachung, d.h. Bestimmung der Regression, Progression, des Verlaufs und/oder des Ausbruchs, von neoplastischen Erkrankungen des Endometriums, insbesondere Tumorerkrankungen des Endometriums und deren Metastasen.

In einem Aspekt betrifft die Erfindung ein Verfahren zur Diagnose und/oder Überwachung eines endometrialen Tumors bei einem Patienten, umfassend (i) den Nachweis und/oder die Bestimmung der Menge einer Nukleinsäure, die ausgewählt ist aus der Gruppe bestehend aus: (a) einer Nukleinsäure, die eine Nukleinsäuresequenz umfasst, die aus der Gruppe bestehend aus SEQ ID NO: 3, 6 und 7 und einem Teil von mindestens 30 aufeinanderfolgenden Nukleotiden davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist, und/oder (ii) den Nachweis und/oder die Bestimmung der Menge eines durch die Nukleinsäure unter (i) kodierten Proteins oder Peptids oder eines Teils davon, in einer aus einem Patienten isolierten biologischen Probe, die endometriales Körpergewebe umfasst, wobei ein Vorliegen der Nukleinsäure, des Proteins oder Peptids oder des Teils davon und/oder eine im Vergleich zu einem Patienten ohne einen endometrialen Tumor, ohne ein Risiko für einen endometrialen Tumor, ohne eine Metastasierung eines endometrialen Tumors und/oder ohne ein Risiko für eine Metastasierung eines endometrialen Tumors erhöhte Menge der Nukleinsäure, des Proteins oder Peptids oder des Teils davon auf das Vorliegen eines endometrialen Tumors, ein Risiko für einen endometrialen Tumor, eine Metastasierung eines endometrialen Tumors und/oder ein Risiko für eine Metastasierung eines endometrialen Tumors hinweist.

In bestimmten Ausführungsformen weist der Patient eine neoplastische Erkrankung auf, steht in Verdacht, an einer neoplastischen Erkrankung erkrankt zu sein oder daran zu erkranken, oder weist ein Risiko für eine neoplastische Erkrankung auf. In weiteren Ausführungsformen weist der Patient eine Metastasierung einer neoplastische Erkrankung auf, steht in Verdacht, an einer Metastasierung einer neoplastischen Erkrankung erkrankt zu sein oder daran zu erkranken, oder weist ein Risiko für eine Metastasierung einer neoplastische Erkrankung auf. In bestimmten Ausführungsformen wurde eine Behandlung einer neoplastischen Erkrankung wie eine Behandlung durch Tumorresektion, Chemo- und/oder Strahientherapie bereits bei dem Patienten durchgeführt oder eine derartige Behandlung ist geplant.

In einem weiteren Aspekt betrifft die Erfindung ein Verfahren zur Bewertung und/oder Prognostizierung des Metastasierungsverhaltens und/oder des Auftretens eines Rezidivs eines endometrialen Tumors, umfassend (i) den Nachweis und/oder die Bestimmung der Menge einer Nukleinsäure, die ausgewählt ist aus der Gruppe bestehend aus: (a) einer Nukleinsäure, die eine Nukleinsäuresequenz umfasst, die aus der Gruppe bestehend aus SEQ ID NO: 3, 6 und 7 und einem Teil von mindestens 30 aufeinanderfolgenden Nukleotiden davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist, und/oder (ii) den Nachweis und/oder die Bestimmung der Menge eines durch die Nukleinsäure unter (i) kodierten Proteins oder Peptids oder eines Teils davon, in einer aus einem Patienten isolierten biologischen Probe, die endometriales Körpergewebe umfasst, wobei ein Vorliegen der Nukleinsäure, des Proteins oder Peptids oder des Teils davon und/oder eine im Vergleich zu einem Patienten ohne einen endometrialen Tumor, ohne ein Risiko für einen endometrialen Tumor, ohne eine Metastasierung eines endometrialen Tumors, ohne ein Risiko für eine Metastasierung eines endometrialen Tumors, ohne ein Rezidiv eines endometrialen Tumors und/oder ohne ein Risiko für ein Rezidiv eines endometrialen Tumors erhöhte Menge der Nukleinsäure, des Proteins oder Peptids oder des Teils davon auf das Vorliegen einer Metastasierung oder eines Rezidivs eines endometrialen Tumors oder ein Risiko für eine Metastasierung oder ein Rezidiv eines endometrialen Tumors hinweist.

In bestimmten Ausführungsformnen weist der Patient eine neoplastische Erkrankung auf, steht in Verdacht, an einer neoplastischen Erkrankung erkrankt zu sein oder daran zu erkranken, oder weist ein Risiko für eine neoplastische Erkrankung auf. In weiteren Ausführungsformen weist der Patient eine Metastasierung einer neoplastische Erkrankung auf, steht in Verdacht, an einer Metastasierung einer neoplastischen Erkrankung erkrankt zu sein oder daran zu erkranken, oder weist ein Risiko für eine Metastasierung einer neoplastische Erkrankung auf. In bestimmten Ausführungsformen wurde eine Behandlung einer neoplastischen Erkrankung wie eine Behandlung durch Tumorresektion, Chemo- und/oder Strahlentherapie bereits bei dem Patienten durchgeführt oder eine derartige Behandlung ist geplant.

Die erfindungsgemäßen Verfahren ermöglichen vorzugsweise eine prognostische Aussage darüber, ob eine Metastasierung eines endometrialen Tumors erfolgt ist oder erfolgen wird. Vorzugsweise erlauben die erfindungsgemäßen Verfahren eine Unterscheidung zwischen benignen und malignen Veränderungen und können Aufschluss über den Erfolg einer bereits erfolgten oder durchzuführenden Behandlung eines endometrialen Tumors wie einer Behandlung durch Tumorresektion, Chemo- und/oder Strahlentherapie geben. Insbesondere können die erfindungsgemäßen Verfahren Aufschluss über die Wahrscheinlichkeit des Auftretens eines Rezidivs bei einer bereits erfolgten oder durchzuführenden Behandlung eines endimetrialen Tumors geben.

Möglichkeiten zur Durchführung eines Nachweises und/oder einer Bestimmung der Menge in den erfindungsgemäßen Verfahren sind dem Fachmann bekannt.

In bestimmten Ausführungsformen umfasst der Nachweis und/oder die Bestimmung der Menge in den erfindungsgemäßen Verfahren (i) die Kontaktierung der biologischen Probe mit einem Mittel, das spezifisch an die Nukleinsäureyan das Protein oder Peptid oder den Teil davon bindet, und (ii) den Nachweis der Komplexbildung zwischen dem Mittel und der Nukleinsäure oder dem Protein oder Peptid oder dem Teil davon_{.}

Ein Nachweis einer Nukleinsäure oder eine Bestimmung der Menge einer Nukleinsäure kann erfindungsgemäß mit einer Oligo- oder Polynukleotid-Sonde erfolgen, die spezifisch mit der Nukleinsäure hybridisiert, oder kann durch selektive Amplifikation, vorzugsweise Amplifikation durch Polymerase-Kettenreaktion, der Nukleinsäure erfolgen. In einer Ausführungsform umfasst die Sonde eine Sequenz von 6-50, insbesondere 10-30, 15-30 oder 20-30 zusammenhängenden Nukleotiden aus der nachzuweisenden Nukleinsäure.

Ein Nachweis eines Proteins oder Peptids oder eines Teils davon oder eine Bestimmung der Menge eines Proteins oder Peptids oder eines Teils davon kann erfindungsgemäß mit einem Antikörper erfolgen, der spezifisch an das Protein oder Peptid oder den Teil davon bindet.

In einer Ausführungsformen liegt das nachzuweisende Protein oder Peptid oder der Teil davon in einem Komplex mit einem MHC-Molekül vor.

Das für einen Nachweis oder für eine Bestimmung der Menge verwendete Mittel, insbesondere die Oligo- oder Polynukleotid-Sonde, oder der Antikörper sind vorzugsweise nachweisbar markiert. In bestimmten Ausführungsformen ist der nachweisbare Marker ein radioaktiver Marker, Fluoreszenzmarker oder Enzymmarker.

Erfindungsgemäß betrifft der Begriff "binden" eine spezifische Bindung. "Spezifische Bindung" bedeutet, dass eine Bindung an ein Ziel wie ein Epitop, für das ein Bindemittel wie ein Antikörper spezifisch ist, stärker ist im Vergleich zu der Bindung an ein anderes Ziel.

Eine ^{"}stärker Bindung" kann beispielsweise durch eine niedrigere Dissoziationskonstante charakterisiert werden.

### Detaillierte Beschreibung der Erfindung

Eine Nukleinsäure ist erfindungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann erfindungsgemäß als einzelsträngiges oder doppelsträngiges und lineares oder kovalent kreisförmig geschlossenes Molekül vorliegen.

Mit "Derivat" einer Nukleinsäure ist gemeint, dass einzelne oder multiple, vorzugsweise mindestens 2, mindestens 4, mindestens 6, und vorzugsweise bis 3, bis 4, bis 5, bis 6, bis 10, bis 15 oder bis 20, Substitutionen, -deletionen und/oder -additionen von Nukleotiden in der Nukleinsäure vorliegen. Weiterhin umfasst der Begriff "Derivat" einer Nukleinsäure auch eine chemische Derivatisierung einer Nukleinsäure an einer Nukleotidbase, am Zucker oder am Phosphat und Nukleinsäuren, die nicht in der Natur vorkommende Nukleotide und Nukleotidanaloga enthalten.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) in *vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989 oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5mM NaH₂PO₄ (pH7), 0,5% SDS, 2mM EDTA). SSC ist 0,15 M Natriumchlorid/ 0,15 M Natriumcitrat, pH 7. Nach der Hybridisierung wird die Membran, auf die die DNA übertragen wurde, beispielsweise in 2 x SSC bei Raumtemperatur und sodann in 0,1 - 0,5 x SSC/ 0,1 x SDS bei Temperaturen bis 68°C gewaschen.

Komplementäre Nukleinsäuren weisen erfindungsgemäß mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Der Begriff "% Identität" soll einen Prozentwert an Nukleotiden bezeichnen, die zwischen zwei zu vergleichenden Sequenzen bei einem optimalen Alignment identisch sind, wobei dieser Prozentwert rein statistisch ist, die Unterschiede zwischen den zwei Sequenzen zufällig und über die gesamte Sequenzlänge verteilt sein können und die zu vergleichende Sequenz Additionen oder Deletionen im Vergleich zu der Referenzsequenz umfassen kann, um ein optimales Alignment zwischen zwei Sequenzen zu erreichen. Sequenzvergleiche zwischen zwei Sequenzen werden im Allgemeinen durch Vergleich dieser Sequenzen nach einem optimalen Alignment in Bezug auf ein Segment oder "Vergleichsfenster" durchgeführt, um lokale Bereiche einer Sequenzübereinstimmung zu identifizieren. Das optimale Alignment für einen Vergleich kann manuell oder mit Hilfe des lokalen Homologiealgorithmus von Smith and Waterman, 1981, Ads App. Math. 2, 482, mit Hilfe des lokalen Homologiealgorithmus von Neddleman and Wunsch, 1970, J. Mol. Biol. 48, 443, und mit Hilfe des Ähnlichkeitssuchalgorithmus von Pearson and Lipman, 1988, Proc. Natl. Acad. Sci. USA 85, 2444, oder mit Hilfe von Computerprogrammen, die diese Algorithmen verwenden, (GAP, BESTFIT, FASTA, BLAST P, BLAST N and TFASTA in Wisconsin Genetics Software Package, Genetics Computer Group, 575 Science Drive, Madison, Wis.) durchgeführt werden.

Die prozentuale Identität wird durch Bestimmung der Anzahl an identischen Positionen, in denen die zu vergleichenden Sequenzen übereinstimmen, Teilung dieser Anzahl durch die verglichenen Positionen und Multiplikation dieses Ergebnisses mit 100 erhalten.

Beispielsweise kann das Programm BLAST "BLAST 2 sequences", das von der Website http://www.ncbi.nlm.nih.gov/blast/b12seq/wblast2.cgi erhältlich ist, verwendet werden.

Nukleinsäuren können alleine oder in Kombination mit anderen Nukleinsäuren, die homo- oder heterolog sein können, vorliegen. In bestimmten Ausführungsformen liegt eine Nukleinsäure funktionell in Verbindung mit Expressionskontrollsequenzen vor, die in Bezug zu der Nukleinsäure homolog oder heterolog sein können, wobei hier der Begriff "homolog" bezeichnet, dass eine Nukleinsäure auch natürlicherweise mit der Expressionskontrollsequenz funktionell verbunden ist und der Begriff "heterolog" bezeichnet, dass eine Nukleinsäure nicht natürlicherweise mit der Expressionskontrollsequenz funktionell verbunden ist.

Eine Nukleinsäure, vorzugsweise eine transkribierbare und insbesondere eine für ein Peptid oder Protein kodierende Nukleinsäure, und eine Expressionskontrollsequenz sind dann "funktionell" miteinander verbunden, falls sie derart kovalent miteinander verknüpft sind, dass die Transkription oder Expression der Nukleinsäure unter der Kontrolle oder unter dem Einfluss der Expressionskontrollsequenz steht. Falls die Nukleinsäure in ein funktionelles Peptid oder Protein translatiert werden soll, führt bei einer funktionellen Verbindung einer Expressionskontrollsequenz mit der kodierenden Sequenz eine Induktion der Expressionskontrollsequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Peptid oder Protein translatiert zu werden.

Der Begriff "ExpressionskontroHsequenz" umfasst Promotoren, ribosombindende Sequenzen und andere Kontrollelemente, welche die Transkription eines Gens oder die Translation der abgeleiteten RNA steuern. In bestimmten Ausführungsformen sind die Expressionskontrollsequenzen regulierbar. Die genaue Struktur von Expressionskontrollsequenzen kann speziesabhängig oder zelltypusabhängig variieren, umfasst jedoch im allgemeinen 5'-nicht-transkribierte und 5'- und 3'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte-Expressionskontrollsequenzen eine Promotorregion, die eine Promotorsequenz für eine transkriptionelle Kontrolle der funktionell verbundenen Nukleinsäure einschließt. Expressionskontrollsequenzen können auch Enhancer-Sequenzen oder stromaufwärts gelegene Aktivatorsequenzen umfassen.

Der Begriff "Promotor" oder "Promotorregion" betrifft eine DNA-Sequenz, die stromaufwärts (5') zu der kodierenden Sequenz eines Gens liegt und die Expression der kodierenden Sequenz durch Bereitstellen einer Erkennungs- und Bindungsstelle für RNA-Polymerase steuert. Die Promotorregion kann weitere Erkennungs- oder Bindungsstellen für weitere Faktoren beinhalten, die an der Regulation der Transkription des Gens beteiligt sind. Ein Promotor kann die Transkription eines prokaryontischen oder eukaryontischen Gens steuern. Ein Promotor kann "induzierbar" sein und die Transkription in Reaktion auf ein Induktionsmittel initiieren oder er kann "konstitutiv" sein, falls die Transkription nicht durch ein Induktionsmittel gesteuert wird. Ein induzierbarer Promotor wird nicht exprimiert oder nur in einem sehr geringen Maß exprimiert, wenn ein Induktionsmittel fehlt. In Gegenwart des Induktionsmittels wird das Gen "angeschalten" oder das Transkriptionsniveau erhöht. Dies wird herkömmlicherweise durch die Bindung eines spezifischen Transkriptionsfaktors vermittelt.

Bevorzugte Promotoren sind beispielsweise Promotoren für SP6-, T3- oder T7-Polymerase.

Der Begriff "Expression" wird in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. In Bezug auf RNA betrifft der Begriff "Expression" oder "Translation" insbesondere die Produktion von Peptiden oder Proteinen.

Des weiteren kann eine Nukleinsäure, die für ein Protein oder Peptid kodiert, Verbindung mit einer anderen Nukleinsäure vorliegen, die für eine Peptidsequenz kodiert, die eine Sekretion des durch die Nukleinsäure kodierten Proteins oder Peptids aus einer Wirtszelle steuert. Auch kann eine Nukleinsäure in Verbindung mit einer anderen Nukleinsäure vorliegen, die für eine Peptidsequenz kodiert, die eine Verankerung des kodierten Proteins oder Peptids auf der Zellmembran einer Wirtszelle oder seine Kompartimentalisierung in bestimmte Organellen dieser Zelle herbeiführt. Gleichermaßen kann eine Verbindung mit einer Nukleinsäure erfolgen, die ein Reportergen oder einen beliebigen "Tag" darstellt.

In einer bevorzugten Ausführungsform ist eine Nukleinsäure in einem Vektor, gegebenenfalls mit einem Promotor, der die Expression der Nukleinsäure steuert, vorhanden. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryotische und/oder in eukaryotische Zellen einzubringen und gegebenenfalls in ein Genom zu integrieren. Solche Vektoren werden vorzugsweise in der Zelle repliziert und/oder exprimiert. Vektoren umfassen Plasmide, Phagemide oder Virusgenome. Der Begriff "Plasmid", wie hierin verwendet, betrifft allgemein ein Konstrukt von extrachromosomalem genetischen Material, gewöhnlich ein zirkuläres DNA-Duplex, das unabhängig von chromosomaler DNA replizieren kann.

Der Begriff "Wirtszelle" betrifft jede Zelle, die mit einer exogenen Nukleinsäure, vorzugsweise DNA oder RNA, transformierbar oder transfizierbar ist. Der Begriff "Wirtszelle" umfasst erfindungsgemäß prokaryontische (z.B. E. *coli)* oder eukaryontische Zellen (z.B. Säugerzellen, insbesondere Zellen aus Mensch, Hefezellen und Insektenzellen). Besonders bevorzugt sind Säugerzellen wie Zellen aus Mensch, Maus, Hamster, Schwein, Ziege und Primaten. Die Zellen können aus einer Vielzahl von Gewebetypen abgeleitet sein und umfassen primäre Zellen und Zelllinien. Spezifische Beispiele umfassen Keratinocyten, periphere Blutleukocyten, Stammzellen des Knochenmarks und embryonale Stammzellen. In weiteren Ausführungsformen ist die Wirtszelle eine Antigen-präsentierende Zelle, wobei der Begriff "Antigen-präsentierende Zelle" erfindungsgemäβ dendritische Zellen, Monocyten und Makrophagen umfasst. Eine Nukleinsäure kann in der Wirtszelle in einer einzigen oder in mehreren Kopien vorliegen und wird in einer Ausführungsform in der Wirtszelle exprimiert.

In den Fällen der Offenbaring in denen ein MHC-Molekül ein Protein oder Peptid präsentiert, kann ein Expressionsvektor auch eine Nukleinsäuresequenz umfassen, die für das MHC-Molekül kodiert. Die Nukleinsäuresequenz, die für das MHC-Molekül kodiert, kann auf demselben Expressionsvektor wie die Nukleinsäure, die für das Protein oder Peptid kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Protein oder Peptid noch das MHC-Molekül exprimiert, können beide dafür kodierenden Nukleinsäuren entweder auf demselben Expressionsvektor oder auf verschiedenen Expressionsvektoren in die Zelle transfiziert werden. Falls die Zelle bereits das MHC-Molekül exprimiert, kann nur die Nukleinsäuresequenz, die für das Protein oder Peptid kodiert, in die Zelle transfiziert werden.

Kits zur Amplifikation einer Nukleinsäure, um dadurch die Nukleinsäure nachzuweisen oder ihre Menge zu bestimmen, umfassen beispielsweise ein Paar von Amplifikationsprimem, die an die zu amplifizierende Nukleinsäure hybridisieren. Die Primer umfassen vorzugsweise eine Sequenz von 6-50, insbesondere 10-30, 15-30 oder 20-30 zusammenhängenden Nukleotiden aus der zu amplifizierenden Nukleinsäure und sind nicht-überlappend, um die Bildung von Primer-Dimeren zu venneiden. Einer der Primer wird an einen Strang der zu amplifizierenden Nukleinsäure hybridisieren und der andere Primer wird an den komplementären Strang in einer Anordnung hybridisieren, die eine Amplifikation der Nukleinsäure erlaubt.

"Antisense-Nukleinsäuren" können zur Regulierung, insbesondere der Reduktion der Expression einer Nukleinsäure verwendet werden. Der Begriff "Antisense-Nukleinsäure" betrifft ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Eine "Antisense-Nukleinsäure," umfasst auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann eine Duplex mit der natürlich vorkommenden mRNA, die ein Peptid oder Protein spezifiziert, eingehen und so eine Translation der mRNA in das Peptid oder Protein verhindern. Eine weitere Möglichkeit ist die Verwendung von Ribozymen zur Inaktivierung einer Nukleinsäure. Bevorzugte Antisense-Oligonukleotide weisen eine Sequenz von 6-50, insbesondere 10-30, 15-30 oder 20-30 zusammenhängenden Nukleotiden aus der Ziel-Nukleinsäure auf und sind vorzugsweise vollständig zu der Ziel-Nukleinsäure oder einem Teil davon komplementär.

Bevorzugt hybridisiert das Antisense-Oligonukleotid mit einer N-terminalen oder 5'-stromaufwärts gelegenen Stelle wie einer Translationsinitiations-, Transkriptionsinitiations- oder Promotorstelle, oder das Antisense-Oligonukleotid hybridisiert mit einer 3'-nicht-translatierten Region oder mRNA-Splicing-Stelle.

In einer Ausführungsform besteht ein Oligonukleotid erfindungsgemäß aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

Bevorzugt ist ein Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet ein Oligonukleotid, bei dem (i) mindestens zwei seiner Nukleotide durch eine synthetische Intemukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer oder mehreren kovalent modifizierten Basen und/oder einem oder mehreren kovalent modifizierten Zuckern. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Der Begriff "Peptid" betrifft Substanzen, die mindestens zwei, mindestens 3, mindestens 4, mindestens 6, mindestens 8, mindestens 10, mindestens 13, mindestens 16, mindestens 20 und vorzugsweise bis 50, 100 oder 150 aufeinander folgende Aminosäuren umfassen, die durch Peptidbindungen miteinander verbunden sind. Der Begriff "Protein" betrifft große Peptide, vorzugsweise Peptide mit mindestens 151 Aminosäuren, jedoch werden die Begriffe "Peptid" und "Protein" hierin im Allgemeinen als Synonyme verwendet.

Die beschriebenen Proteine und Peptide sind vorzugsweise isoliert. Die Begriffe "isoliertes Protein" oder "isoliertes Peptid" bedeuten, dass das Protein oder Peptid von seiner natürlichen Umgebung getrennt ist. Ein isoliertes Protein oder Peptid kann in einem im Wesentlichen aufgereinigten Zustand vorliegen. Der Begriff "im Wesentlichen aufgereinigt" bedeutet, dass das Protein oder Peptid im Wesentlichen frei von anderen Substanzen vorliegt, mit denen es in der Natur oder in *vivo* vorliegt.

Solche Proteine und Peptide dienen beispielsweise der Herstellung von Antikörpern und sind in einem immunologischen oder diagnostischen Assay oder als Therapeutika einsetzbar. Hier beschriebene Proteine und Peptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Peptids oder einer Aminosäuresequenz umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Peptiden nicht konserviert sind und/oder werden Aminosäuren durch andere mit ähnlichen Eigenschaften ersetzt, wie Hydrophobizität, Hydrophilizität, Elektronegativität, Volumen der Seitenkette und ähnliches (konservative Substitution). Konservative Substitutionen betreffen beispielsweise den Austausch einer Aminosäure durch eine andere, nachstehend in derselben Gruppe wie die substituierte Aminosäure aufgeführte Aminosäure:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Festphasensynthese" (Merrifield, 1964) und ähnliche Verfahren oder durch rekombinante DNA-Manipulation hergestellt werden. Die Manipulation von DNA-Sequenzen zur Herstellung von Proteinen und Peptiden mit Substitutionen, Insertionen oder Deletionen ist z.B. in Sambrook et. al. (1989) ausführlich beschrieben.

"Derivate" von Proteinen oder Peptiden umfassen auch einzelne oder multiple Substitutionen, Deletionen und/oder Additionen jeglicher Moleküle, die mit dem Protein oder Peptid assoziiert sind, wie Kohlenhydrate, Lipide und/oder Proteine oder Peptide. Ferner erstreckt sich der Begriff "Derivat" auch auf alle funktionellen chemischen Äquivalente der Proteine und Peptide und Substanzen, die nicht nur Aminosäure-Bestandteile, sondern auch nicht-Aminosäure-Bestandteile wie Zucker und Phosphatstrukturen enthalten und umfassen auch Substanzen die Bindungen wie Ester-, Thioether- oder Disulfidbindungen enthalten.

Ein Teil oder Fragment eines Proteins oder Peptids weist vorzugsweise eine funktionelle Eigenschaft des Proteins oder Peptids auf, aus dem es abgeleitet ist. Solche funktionellen Eigenschaften umfassen beispielsweise die Interaktion mit Antikörpern oder die Interaktion mit anderen Peptiden oder Proteinen. Eine bedeutende Eigenschaft ist die Fähigkeit, einen Komplex mit MHC-Molekülen einzugehen und gegebenenfalls eine Immunreaktion beispielsweise durch Stimulation von cytotoxischen oder Helfer T-Zellen zu erzeugen. Vorzugsweise umfasst ein Teil oder Fragment eines Proteins oder Peptids eine Sequenz von mindestens 6, mindestens 8, mindestens 10, mindestens 12, mindestens 15, mindestens 20, oder mindestens 30 und vorzugsweise bis 8, 10, 12, 15, 20, 30 oder 50 aufeinanderfolgenden Aminosäuren aus dem Protein oder Peptid.

Ein Teil oder ein Fragment einer Nukleinsäure, die für ein Protein oder Peptid kodiert, betrifft erfindungsgemäß vorzugsweise den Teil der Nukleinsäure, der zumindest für das Protein oder Peptid und/oder für einen Teil oder ein Fragment des Proteins oder Peptids wie vorstehend definiert kodiert.

Antiseren, die Antikörper enthalten, die an ein Ziel spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von Antikörpern von Bedeutung, die therapeutisch eingesetzt werden sollen, aber auch für viele diagnostische Anwendungen. Dazu kann sowohl mit dem gesamten Protein, mit extrazellulären. Teilsequenzen, wie auch mit Zellen, die das Zielmolekül in physiologisch gefalteter Form exprimieren, immunisiert werden.

Monoklonale Antikörper werden traditionell mit Hilfe der Hybridoma-Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447).

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Deteminanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Als anderes Beispiel beschreibt die WO 92/04381 die Herstellung und Verwendung von humanisierten RSV-Antikörpem aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Der Begriff "Antikörper" umfasst auch F(ab')₂-, Fab-, Fv- und Fd-Fragmente von Antikörpern, chimäre Antikörper, bei denen die Fc- und/oder FR- und/oder CDR1- und/oder CDR2- und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden. Der Begriff "Antikörper" umfasst auch einzelkettige Antikörper.

Antikörper können auch an spezifische diagnostische Mittel gekoppelt werden, um beispielsweise Zellen und Gewebe darzustellen, die bestimmte Proteine oder Peptide exprimieren. Sie können ferner an therapeutische Mittel gekoppelt werden.

Diagnostische Mittel umfassen eine jegliche Markierung, die geeignet ist: (i) ein nachweisbares Signal bereitzustellen, (ii) mit einer zweiten Markierung wechselzuwirken, um das nachweisbare Signal, das durch die erste oder zweite Markierung bereitgestellt wird, zu modifizieren, z.B. FRET (Fluoreszenz-Resonanz-Energietransfer), (iii) die Mobilität wie elektrophoretische Mobilität durch Ladung, Hydrophobizität, Form oder andere physikalische Parameter zu beeinflussen oder (iv) eine Abfanggruppe bereitzustellen, z.B. Affinitäts-, Antikörper/Antigen- oder ionische Komplexierung. Geeignet als Markierungen sind Strukturen wie Fluoreszenzmarkierungen, Lumineszenzmarkierungen, Chromophormarkierungen, Radioisotopenmarkierungen, Isotopenmarkierungen, vorzugsweise stabile Isotopenmarkierungen, Enzymmarkierungen, Partikelmarkierungen, insbesondere Metallpartikelmarkierungen, Magnetpartikelmarkierungen, Polymerpartikelmarkierungen, kleine organische Moleküle wie Biotin, Liganden von Rezeptoren oder Bindemolekülen wie Zelladhäsionsproteine oder Lektine, und Markierungssequenzen, die Nukleinsäure- und/oder Aminosäuresequenzen umfassen. Diagnostische Mittel umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff- 11, gamma-Emitter wie Iod-123, Technetium-99m, Iod-131 und Indium-111, und Nuklide für magnetische Kernresonanz wie Fluorin und Gadolinium.

Der Begriff "therapeutisches Mittel" betrifft erfindungsgemäß jede Substanz, die eine therapeutische Wirkung ausüben kann und umfasst in nicht begrenzender Weise Antikrebsmiael, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabidin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner)) beschrieben. Toxine können Proteine wie Pokeweed-antivirales Protein, Choleratoxin, Pertussistoxin, Ricin, Gelonin, Abrin, Diphtherie-Exotoxin oder *Pseudomonas*-Exotoxin sein. Toxinreste können auch Hochenergie-emittierende Radionuklide wie Kobalt-60 sein.

Der Begriff "Haupthistokompatibilitätskomplex" oder "MHC" betrifft einen Komplex von Genen, der in allen Vertebraten vorhanden ist. MHC-Proteine oder Moleküle sind bei der Signalgebung zwischen Lymphocyten und Antigen-präsentierenden Zellen in normalen Immunreaktionen beteiligt, wobei sie Peptide binden und diese für eine Erkennung durch T-Zell-Rezeptoren präsentieren. MHC-Moleküle binden Peptide innerhalb eines intrazellulären Prozessierungskompartiments und präsentieren diese Peptide auf der Oberfläche von Antigen-präsentierenden Zellen für eine Erkennung durch T-Zellen. Die menschliche MHC-Region, auch HLA genannt, liegt auf Chromosom 6 und umfasst die Klasse I- und Klasse II-Region. In einer bevorzugten Ausführungsform gemäß aller erfindungsgemäßer Aspekte ist ein MHC-Molekül ein HLA-Molekül.

Der Begriff "Patient" umfasst erfindungsgemäß männliche und weibliche Patienten, vorzugsweise weibliche Patienten. Beispiele von Patienten umfassen erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "neoplastische Erkrankung" betrifft eine Neubildung von Körpergeweben im Sinne eines dysregulierten, enthemmten und/oder autonomen Überschußwachstums, wobei der Begriff "Erkrankung" einen jeglichen pathologischen Zustand betrifft. Eine neoplastische Erkrankung ist eine Tumor- oder Krebserkrankung wie Leukämien, Seminome, Melanome, Teratome, Gliome, Nieren-, Nebennieren-, Schilddrüsen-, Darm-, Leber-, Colon-, Magen-, Gastrointestinal-, Lymphknoten-, Speiseröhren-, Kolorektal-, Pankreas-, Hals, Nasen, Ohren (HNO)-, Brust-, Prostata-, Gebärmutter-, Ovarial-, Knochen-, Vaginal- und Lungenkrebs und insbesondere Krebs des Endometriums und deren Metastasen, die bei Krebs des Endometriums insbesondere in para-aortalen Lymphknoten, Knochen, Lunge, Becken, Leber und Vagina auftreten. Bevorzugt ist eine neoplastische Erkrankung durch eine Karzinogenese induziert. Neoplasien betreffen erfindungsgemäß benigne (gutartige) Veränderungen ohne Metastasierung und maligne (bösartige) Veränderungen, insbesondere mit einem infiltrierendem Wachstum und unter Bildung von Metastasen.

Der Begriff "Myometrium" betrifft erfindungsgemäß die von der glatten Muskulatur gebildete kräftige Mittelschicht der Uteruswand.

Der Begriff "Rezidiv" betrifft erfindungsgemäß einen Rückfall einer Erkrankung, insbesondere ihr Wiederauftreten nach einer Abheilung oder scheinbaren Abheilung. In Bezug auf eine Tumorerkrankung betrifft der Begriff "Rezidiv" das Wiederauftreten von Tumoren nach zunächst erfolgreicher Behandlung wie Behandlung durch Operation, Chemo- und/oder Strahlentherapie.

Der Begriff "erhöhte Menge" betrifft vorzugsweise eine Erhöhung um mindestens 10%, insbesondere mindestens 20%, mindestens 50% oder mindestens 100%. Die Menge einer Substanz ist auch dann in einem Testobjekt wie einer biologischen Probe in Bezug auf eine Referenz erhöht, wenn sie in dem Testobjekt nachweisbar ist, jedoch in der Referenz nicht vorhanden und/oder nicht nachweisbar ist.

Eine biologische Probe kann eine Gewebeprobe, einschließlich Körperflüssigkeiten, und/oder eine zelluläre Probe sein und kann in herkömmlicher Weise gewonnen werden, wie durch Gewebebiopsie, einschließlich Stanzbiopsie, und Entnahme von Blut, Bronchialaspirat, Sputum, Urin, Fäces oder anderen Körperflüssigkeiten.

Die Begriffe "T-Zelle" und "T-Lymphocyt" umfassen T-Helferzellen und cytolytische oder cytotoxische T-Zelien.

Manche therapeutische Verfahren beruhen auf einer Reaktion des Immunsystems eines Patienten, die zu einer Lyse Antigen-präsentierender Zellen führt, wie Krebszellen, die ein oder mehrere Peptide präsentieren. Dabei werden beispielsweise autologe cytotoxische T-Lymphocyten, die für einen Komplex aus einem Peptid und einem MHC-Molekül spezifisch sind, an einen Patienten mit einer Zellabnormalie verabreicht. Die Produktion solcher cytotoxischer T-Lymphocyten *in vitro* ist bekannt.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989)_{,} werden Zellen, die den gewünschten Komplex präsentieren (z.B. dendritische Zellen) mit cytotoxischen T-Lymphocyten des zu behandelnden Patienten kombiniert, was zu einer Vermehrung spezifischer cytotoxischer T-Lymphocyten führt. Die vermehrten cytotoxischen T-Lymphocyten werden sodann an einen Patienten mit einer zellulären Abnormalie verabreicht, wobei die abnormalen Zellen den spezifischen Komplex präsentieren. Die cytotoxischen T-Lymphocyten lysieren sodann die abnormalen Zellen, wodurch eine gewünschte therapeutische Wirkung erreicht wird.

Der adoptive Transfer ist nicht die einzige Therapieform, die anwendbar ist. Cytotoxische T-Lymphocyten können auch *in* vivo in an sich bekannter Weise erzeugt werden. Bei einem Verfahren werden nicht-proliferative Zellen verwendet, die den Komplex exprimieren wie bestrahlte Tumorzellen oder Zellen, die mit einem oder beiden Genen transfiziert wurden, die für eine Präsentation des Komplexes notwendig sind (d.h. das antigene Peptid und das präsentierende MHC-Molekül). Eine bevorzugte Form ist die Einbringung eines Proteins oder Peptids, das für einen Tumor kennzeichnend ist, in Form von rekombinanter RNA in Zellen, die sodann den interessierenden Komplex präsentieren. Solche Zellen werden von autologen cytotoxischen T-Lymphocyten erkannt, die sich sodann vermehren.

Eine ähnliche Wirkung kann durch Kombination eines Proteins oder Peptids mit einem Adjuvans erreicht werden, um einen Einbau in Antigen-präsentierende Zellen *in vivo* zu ermöglichen. Das Protein oder Peptid kann als solches, als DNA (z.B. innerhalb eines Vektors) oder als RNA repräsentiert sein. Das Protein oder Peptid kann prozessiert werden, um einen Peptidpartner für das HLA-Molekül zu ergeben. Eine Präsentation ist auch möglich, ohne dass eine weitere Prozessierung erforderlich ist. Dies ist insbesondere der Fall, wenn Peptide an HLA-Moleküle binden können. Verabreichungsformen, bei denen das Gesamt-Antigen in *vivo* von einer dendritischen Zelle prozessiert wird, sind bevorzugt, da hier auch Helfer T-Zell-Antworten entstehen können, die für eine effektive Immunantwort benötigt werden (Ossendorp et al., Immunol Lett. 74:75-9, 2000; Ossendorp et al., J. Exp. Med. 187:693-702, 1998).

Die Begriffe "Immunisierung" oder "Vakzinierung" bedeuten eine Erhöhung oder Aktivierung einer Immunreaktion gegenüber einem Antigen. Tiermodelle können zum Testen einer immunisierenden Wirkung gegenüber Krebs eingesetzt werden. Zum Beispiel können menschliche Krebszellen in eine Maus für die Schaffung eines Tumors eingebracht werden und eine oder mehrere Nukleinsäuren, die für Proteine oder Peptide, die für Krebszellen charakteristisch sind, kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden vorzugsweise ein oder mehrere Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und/oder Stimulierung bestimmter Lymphocyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Kreig et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Cytokine bei einer Vakzinierung aufgrund ihrer regulatorischen Eigenschaften auf Lymphocyten verwendbar. Solche Cytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Eine Verabreichung von Proteinen und Peptiden kann in an sich bekannter Weise erfolgen. Beispielsweise erfolgt die Verabreichung von Nukleinsäuren durch ex *vivo*-Verfahren, d.h. durch Entfernung von Zellen aus einem Patienten, genetische Veränderung der Zellen, um eine Nukleinsäure einzubringen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Erfindungsgemäß vorgesehen ist auch eine Verabreichung von Nukleinsäuren *in vivo* durch die Verwendung von Vektoren wie Viren und zielgesteuerten Liposomen.

Bevorzugt ist ein viraler Vektor für die Verabreichung einer Nukleinsäure aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virus-ähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um Nukleinsäuren in Zellen *in vitro* oder *in vivo* einzubringen. Solche Verfahren umfassen die Transfektion von Nukleinsäure-Calciumphosphat-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Falls eine Verabreichung einer Nukleinsäure durch Liposomen erwünscht ist, können Proteine, die an ein Oberflächenmembran-Protein binden, das mit der Endocytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich, die ebenfalls erfindungsgemäß umfasst sind.

### Abbildungen:

### Abb. 1. Northern Blot-Analyse unter Verwendung einer für EDI-3 spezifischen Sonde.

RNA wurde aus Testis, Skelettmuskel, Leber, Lunge, Milz, Gehirn und Herz (Spuren 1-8) erhalten. Expression von EDI-3-Transkript wurde in Testis (Spur 1), Skelettmuskel (Spur 3) und Herz (Spur 8) festgestellt.

### Abb. 2. Expression von EDI-3 in primären Endometriumkarzinomen.

Eine 6,4-fach höhere Expression wurde in metastasierenden Tumoren im Vergleich zu nicht-metastasierenden Tumoren festgestellt (p<0,001; Mann-Whitney-Test, zweiseitig). Lediglich Patienten mit einem Beobachtungszeitraum von mindestens 5 Jahren wurden eingeschlossen. Jedoch wird auch ein Unterschied hinsichtlich der Expression von EDI-3 erhalten, wenn alle 57 Patienten, einschließlich auch derjenigen mit kürzeren Beobachtungszeiträumen als 5 Jahre, eingeschlossen werden (p<0,001, Daten nicht gezeigt). Die horizontale Linie in der Mitte eines Kästchens zeigt den Medianwert der Probe. Die Kanten eines Kästchens markieren die 25. und 75. Percentile. Die Whisker zeigen den Bereich von Werten, die innerhalb 1,5 Kästchen-Längen liegen.

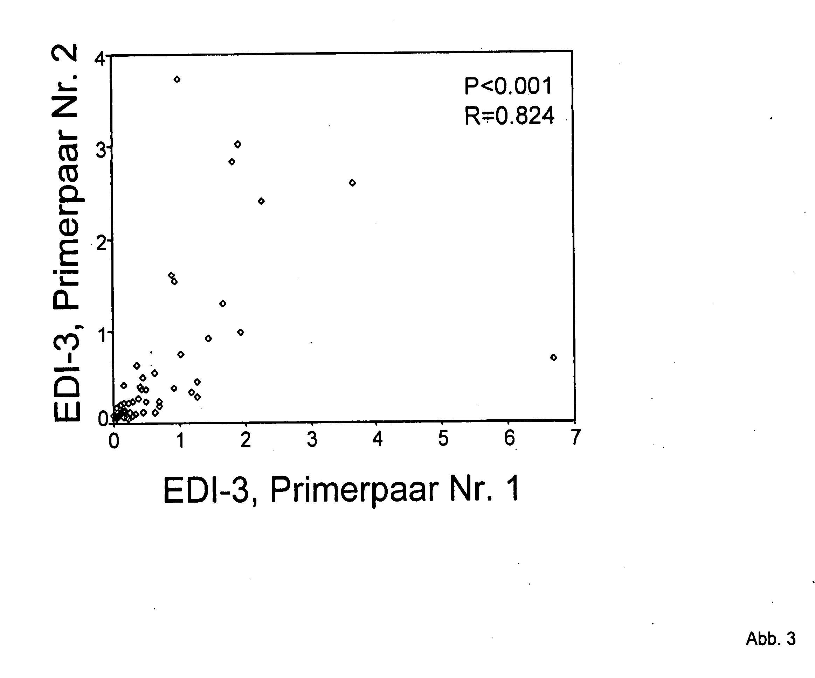

### Abb. 3. Bestätigungsexperiment unter Verwendung eines zweiten Primerpaars zur Quantifizierung der Expression von EDI-3-mRNA.

Primerpaar Nr. 1 amplifiziert ein Fragment zwischen den bp-Positionen 2872 und 3172, während Primerpaar Nr. 2 zu einer Amplifikation zwischen den bp-Positionen 3161 und 3362 führt. Quantitative PCR führte zu einer Korrelation mit p<0,001 und R=0.824.

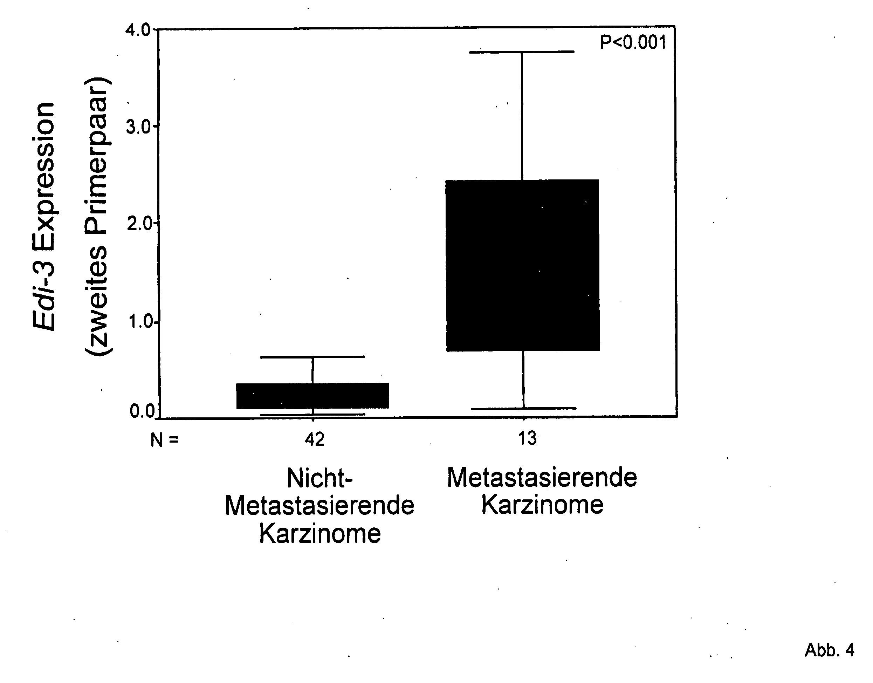

### Abb. 4. Bestätigungsexperiment unter Verwendung eines zweiten Primerpaars zur Quantifizierung der Expression von EDI-3-mRNA.

Ähnlich zu den Ergebnissen, die mit dem ersten Primerpaar erhalten wurden, wurde eine höhere Expression von EDI-3 in metastasierenden Tumoren im Vergleich zu nicht-metastasierenden Tumoren festgestellt (p<0,001, Mann-Whitney-Test, zweiseitig). Nur Patienten mit einem Untersuchungszeitraum von mindestens fünf Jahren wurden berücksichtigt. Die horizontale Linie in der Mitte eines Kästchens zeigt den Medianwert der Probe. Die Kanten eines Kästchens markieren die 25. und 75. Percentile. Die Whisker zeigen den Bereich von Werten, die innerhalb 1,5 Kästchen-Längen liegen.

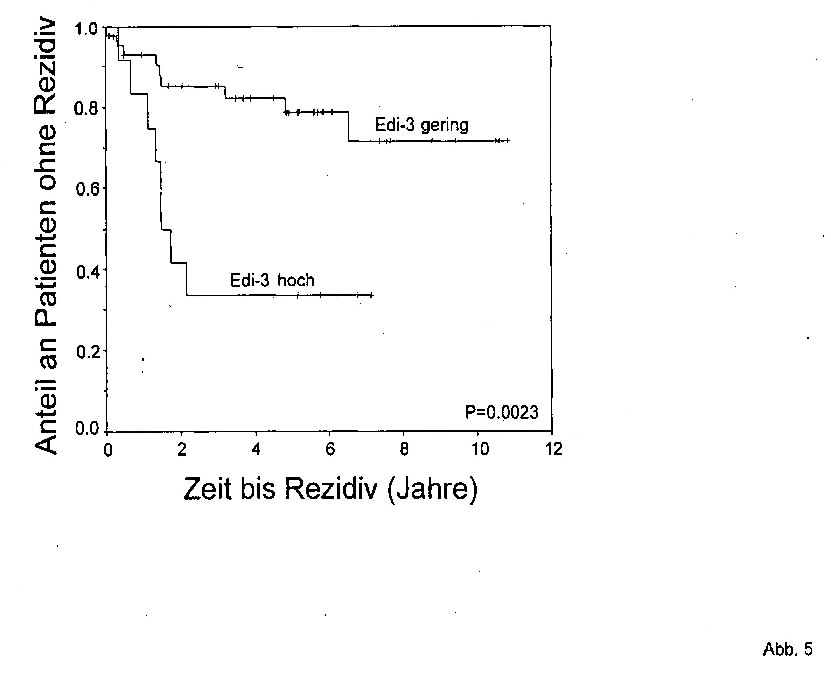

### Abb. 5. Kaplan-Meier-Analyse der Assoziierung zwischen der Expression des EDI-3-Transkripts und der Zeitspanne bis zu einem Rezidiv bei Resektion von endometrialem Krebsgewebe.

Die Expression von EDI-3 wurde unter Verwendung der 75%-Perzentile dichotomisiert (p=0,0023, Logrank-Test).

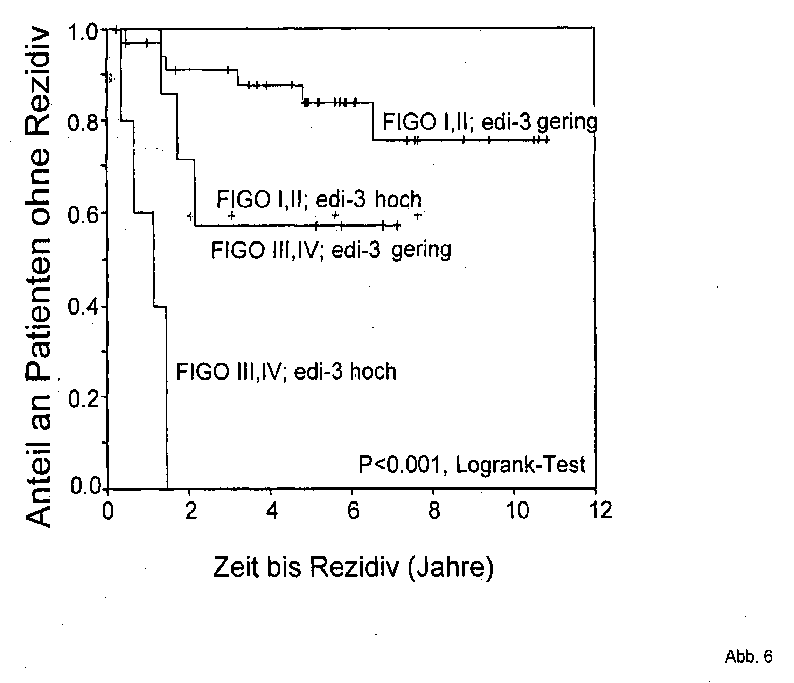

**Abb. 6****. Kaplan-Meier-Analyse der Assoziierung zwischen der Expression des EDI-3-Transkripts und der Zeitspanne bis zu einem Rezidiv abhängig von dem FIGO-Stadium.**

Expression von EDI-3 wurde unter Verwendung der 75%-Perzentile dichotomisiert.

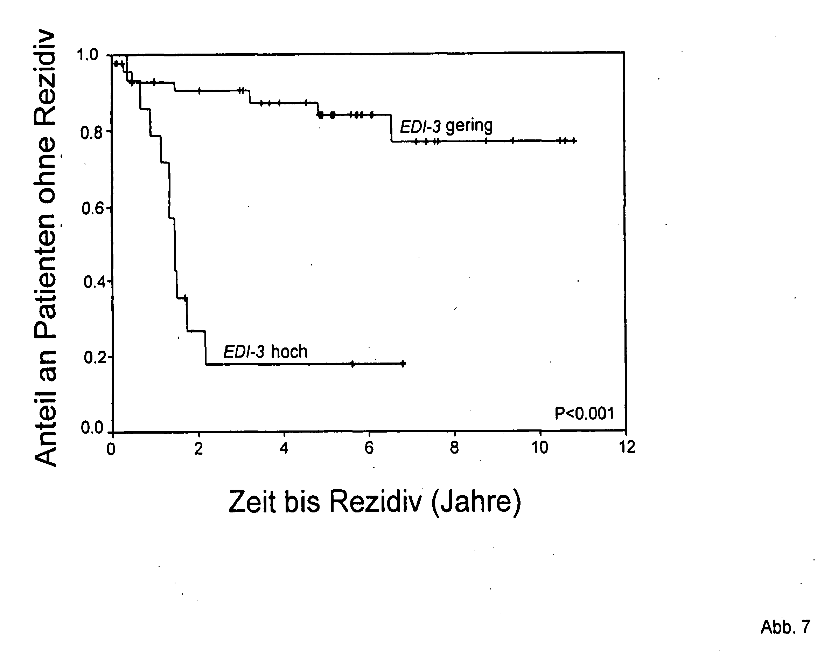

### Abb. 7. Bestätigungsexperiment unter Verwendung eines zweiten Primerpaars zur Quantifizierung der Expression von EDI-3-mRNA.

Ähnlich zu den Ergebnissen, die mit dem ersten Primerpaar erhalten wurden, war die Zeitspanne bis zum Auftreten eines Rezidivs für Patienten mit niedriger Expression von EDI-3 länger im Vergleich zu Patienten mit hoher Expression von EDI-3 bei Resektion von endometrialem Krebsgewebe. EDI-3 wurde bei der 75%-Perzentile dichotomisiert (p<0,001, Logranh-Test).

### Beispiele:

### Beispiel 1: Patienten und Gewebeproben

Zwischen 1985 und 2000 wurden 269 Patienten mit histologisch erwiesenem Krebs des Endometriums in der Abteilung für Geburtshilfe und Frauenheilkunde an der Universitätsklinik in Mainz behandelt.

RNA mit hoher Qualität war nur von 63 dieser Patienten aus drei Gründen erhältlich: (i) Teile der Tumorproben, die für eine RNA-Analyse verwendet wurden, wurden zusätzlich histologisch untersucht. Falls die Fraktion an Tumorzellen geringer als 95% war, wurde die Probe nicht in die vorliegende Untersuchung eingeschlossen. (ii) Kein Gewebe konnte aus manchen Patienten mit kleinen Tumoren eingefroren werden. (iii) Die Qualität mancher RNA-Proben war aufgrund des Verhältnisses von 28S- und 18S-Banden nicht ausreichend oder die Expression des konstitutiven Gens huPO (menschliches Phosphoprotein) war zu gering (Mohrmann G et al., Int. J. Cancer, in press, 2005). Aufgrund von Information aus Klinikaufzeichnungen, einschließlich Operationsberichten und pathologischen Berichten, wurde eine Datenbank erzeugt. Der histologische Tumortyp und -Grad, das Gewicht, die Größe und das Alter der Patienten, Diabetes mellitus, das FIGO-Stadium, der Typ einer Operation und die pathologische TNM-Klassifizierung wurden eingeschlossen. Das FIGO-Stadium folgte dem operativen Stadienbestimmungssystem für endometriale Karzinome von 1988 (Creasman WT, Gynecol Oncol 1989;35: 125-7). Der Körpermassenindex (BMI) wurde unter Verwendung der Formel BMI=[Gewicht/(Größe)²] berechnet. Die Rezidiv-freie Zeit wurde als die Differenz zwischen dem Datum einer operativen Behandlung und dem Datum einer Dokumentation eines Rezidivs berechnet. Rezidive entstanden aufgrund eines neuen Tumorwachstums in para-aortalen Lymphknoten, Becken, Knochen, Lunge, Leber und Vagina. Eine Bewertung aller histologischer Proben erfolgte durch einen erfahrenen Pathologen. Alle Tumoren wurden gemäß der WHO/ISGPY-Klassifizierung klassifiziert (Scully RE et al., International Classification and Histologic Typing of Female Genital Tract Tumours. Springer: New York, 1994). Der Tumorgrad wurde gemäß Kurman et al. (Kurman RJ et al., In: Kurman RJ, ed. Blaustein's Pathology of the Female Genital Tract, 4th edn. Springer: New York. 1994, 439-86) unter Einbeziehung struktureller Merkmale und Kemmerkmale bestimmt. Die Invasionstiefe wurde gemäß Sevin und Angioli (Sevin B-U, Angioli R. Uterine Corpus: Multimodality Therapy in Gynecologic Oncology. Thieme: New York, 1996) abhängig von der Infiltration des inneren, mittleren und äußeren Drittels des Myometriums klassifiziert. Für eine RNA-Isolierung wurden nur histologisch kontrollierte Tumorproben mit mindestens 95% Tumorzellen ohne nicht-neoplastisches Endometrium oder Myometrium in die Untersuchung einbezogen. Ein Standard-Operationsverfahren war eine abdominale Hysterektomie und eine bilaterale Salpingoovariektomie. Eine Lymphknoten-Dissektion erfolgte in Fällen, bei denen intraoperative gefrorene Schnitte eine Infiltration des äußeren Drittels des Myometriums zeigten und auch in Fällen mit einer zervikalen Beteiligung, abhängig von Faktoren einer allgemeinen Morbidität des Patienten.

Unter den 63 Patienten mit verfügbarer RNA einer hohen Qualität wurden drei Patientenpaare mit einem identischen FIGO-Stadium, Grad, histopathologischem Tumortyp, Typ einer Operation, die Menopause betreffenden Zustand, Tiefe einer Invasion in das Myometrium sowie einem ähnlichen Körpermassenindex ausgewählt. Diese Paare bestanden jeweils aus einem Patienten, der innerhalb von fünf Jahren nach einer Operation Metastasen entwickelte und einem weiteren Patienten, bei dem innerhalb eines Beobachtungszeitraums von mindestens fünf Jahren keine Metastasen festgestellt wurden. Die sechs zusammengehörigen Patienten ("Screeningsatz von Tumoren") wurden verwendet, um differentiell exprimierte Gene zu identifizieren.

Die verbleibenden 57 Patienten dienten als "Validierungssatz" und bestanden aus 13 Patienten, die später Metastasen entwickelten, während die anderen Patienten tumorfrei blieben. Der "Validierungssatz" wurde verwendet, um zwei Fragen bezüglich Kandidatengenen, die in dem "Screeningsatz" von Tumoren identifiziert worden waren, anzugehen: (i) Zeigten Primärtumore von Patienten, die später Metastasen entwickelten, eine höhere Expression eines Kandidatengens als Patienten ohne Metastasen? (ii) Wurden Kandidatengene in dem "Screeningsatz von Tumoren" identifiziert, die mit dem Zeitraum bis zu einem Rezidiv in einer multivariaten Analyse assoziiert waren? Die Patientencharakteristika sind in Tabelle 1 zusammengefasst.

**Tabelle 1. Eigenschaften des "Validierungssatzes" von Patienten mit primären emdometrialen Karzinomen.**

| Primäre endometriale Karzinome (n=57) | | | |
|---|---|---|---|
| | Anzahl bewertet (n=57) | % | Nicht auswertbar |
| **FIGO-Stadium** | | | 1 |
| Stadium I | 35 | 62,5 | |
| Stadium II | 7 | 12,5 | |
| Stadium III | 11 | 19,6 | |
| Stadium IV | 3 | 5,4 | |
| | | | |

| **Histologischer Grad** | | | 2 |
|---|---|---|---|
| Grad I | 18 | 32,7 | |
| Grad II | 23 | 41,8 | |
| Grad III | 14 | 25,5 | |
| | | | |

| **Invasionstiefe¹** | | | 0 |
|---|---|---|---|
| gering | 21 | 36,8 | |
| stark | 36 | 63,2 | |
| | | | |

| **Metastasierung²** | | | 2 |
|---|---|---|---|
| Nein | 42 | 76,4 | |
| Ja | 13 | 23,6 | |

| **Status der Menopause** | | | 0 |
|---|---|---|---|
| Pre | 6 | 10,5 | |
| Post | 51 | 89,5 | |
| | | | |

| **Alter bei Operation** | | | |
|---|---|---|---|
| (Jahre, Mittelwert ± Standardabweichung) | 68,5 ± 11,5 | | |
| | | | |

| **Körpergröße** | | | |
|---|---|---|---|
| (cm, Mittelwert ± Standardabweichung) | 163 ± 5,2 | | |
| | | | |

| **Gewicht** | | | |
|---|---|---|---|
| (kg, Mittelwert ± Standardabweichung) | 79,5 ± 16,9 | | |

| | | | |
|---|---|---|---|
| ¹Invasionstiefe wurde als gering (Infiltration maximal des inneren Drittels des Myometriums) und stark (Infiltration des mittleren oder äußeren Drittels des Myometriums) klassifiziert. ²Metastasierung: Nach Entfernen des Primärtumors durch eine Standard-Operation (abdominale Hysterektomie und bilaterale Salpingoovariektomie) wurden zwei Klassen unterschieden: (i) "Keine Metastasierung", falls der Patient tumorfrei blieb, (ii) "Metastasierung" falls erneutes Tumorwachstum an einer der folgenden Stellen festgestellt wurde: para-aortale Lymphknoten, Becken, Knochen, Lunge, Leber oder Vagina. | | | |

### Beispiel 2: Differentialdarstellung

RNA aus gefrorenem Gewebe wurde unter Verwendung eines käuflich verfügbaren Kits (MidiKit, Qiagen, Hilden, Deutschland) isoliert. Die Qualität der isolierten RNA wurde durch das Verhältnis der 28S- und 18S-Banden auf einem 1%-Agarosegel und durch Expression des konstitutiven Gens huPO, wie vorher beschrieben, bewertet (Mohrmann G et al., Int. J. Cancer, in press, 2005). Die Konzentrationen der isolierten RNA wurden spektrophotometrisch bestimmt. Eine reverse Transkription erfolgte unter Verwendung des Delta™ Differential Display Kit (Clontech, Heidelberg, Deutschland). Eine Amplifizierung wurde unter Verwendung der nachstehend gezeigten P- und T-Primer erreicht (Arbitrary Primer, Clontech, Heidelberg, Deutschland):

### P-Primer

P 1: 5'-ATTAACCCTCACTAAATGCTGGGGA-3'
P 2: 5'-ATTAACCCTCACTAAATCGGTCATAG-3'
P 3: 5'-ATTAACCCTCACTAAATGCTGGTGG-3'
P 4: 5'-ATTAACCCTCACTAAATGCTGGTAG-3'
P 5: 5'-ATTAACCCTCACTAAAGATCTGACTG-3'
P 6: 5'-ATTAACCCTCACTAAATGCTGGGTG-3'
P 7: 5'-ATTAACCCTCACTAAATGCTGTATG-3'
P 8: 5'-ATTAACCCTCACTAAATGGAGCTGG-3'
P 9: 5'-ATTAACCCTCACTAAATGTGGCAGG-3'
P 10: 5'-ATTAACCCTCACTAAAGCACCGTCC-3'

### T-Primer

T 1: 5'-CATTATGCTGAGTGATATCTTTTTTTTTAA-3'
T 2: 5'-CATTATGCTGAGTGATATCTTTTTTTTTAC-3'
T 3: 5'-CATTATGCTGAGTGATATCTTTTTTTTTAG-3'
T 4: 5'-CATTATGCTGAGTGATATCTTTTTTTTTCA-3'
T 5: 5'-CATTATGCTGAGTGATATCTTTTTTTTTCC-3'
T 6: 5'-CATTATGCTGAGTGATATCTTTTTTTTTCG-3'
T 7: 5'-CATTATGCTGAGTGATATCTTTTTTTTTGA-3'
T 8: 5'-CATTATGCTGAGTGATATCTTTTTTTTTGC-3'
T 9: 5'-CATTATGCTGAGTGATATCTTTTTTTTTGG-3'

Die Produkte der ersten Amplifizierung wurden auf 8%-igen denaturierenden Polyacrylamidgelen aufgetrennt und durch Silberfärbung unter Verwendung des Rapid-Silver Stain Kit (ICN Biomedicals, Ohio, USA) sichtbar gemacht. Banden, die in Proben von Patienten mit Metastasen erkennbar waren, jedoch bei Proben von Patienten mit nicht-metastasierenden Tumoren fehlten, wurden ausgeschnitten und die DNA wurde unter Verwendung des QiaExIIKit (Qiagen, Hilden, Deutschland) aufgereinigt. Um genug DNA für eine Sequenzierung herzustellen, wurde das aufgereinigte Produkt erneut mit Hilfe der gleichen Primer, die für die Identifizierung verwendet wurden, amplifiziert. Nach Aufreinigung durch QIAquick-Säulen (Qiagen, Hilden, Deutschland) wurde die DNA-Sequenz durch cyclische Sequenzierung bestimmt.

Eine Differentialdarstellungsuntersuchung des "Screeningsatzes von Tumoren" führte zu der Identifizierung von drei Transkripten, nämlich *EDI-1, EDI-2* und *EDI-3,* die in Tumoren vorhanden waren, die Metastasen bildeten, jedoch nicht in nicht-metastasierenden endometrialen Karzinomen exprimiert wurden.

EDI-1 wurde mittels der Clontech Primer P 3 und T 3 aufgefunden und hatte im Polyacrylamidgel eine Länge von 260 bp. Nach Reamplifikation, Aufreinigung und Sequenzierung mit Hilfe des Primers P 3 wurde die in SEQ ID NO:1 gezeigte Nukleinsäuresequenz erhalten.

EDI-2 wurde mittels der. Clontech Primer P 2 und T 5 aufgefunden und wies im Polyacrylamidgel eine Länge von 190 bp auf. Nach Reamplifikation, Aufreinigung und Sequenzierung mit Hilfe des Clontech Primers P 2 wurde die in SEQ ID NO:2 gezeigte Nukleinsäuresequenz erhalten.

EDI-3 wurde mittels der Clontech Primer P 3 und T 3 erhalten und wies im Polyacrylamidgel eine Länge von 270 bp auf. Nach Reamptifkation, Aufreinigung und Sequenzierung mit Hilfe des Clontech Primers P 3 wurde die in SEQ ID NO:3 gezeigte Nukleinsäuresequenz erhalten.

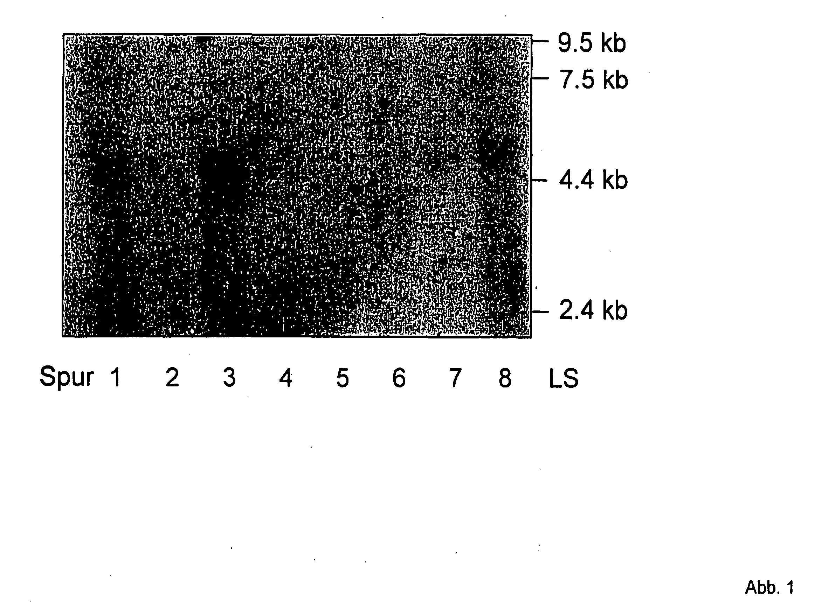

Das sequenzierte Nukleinsäurefragment von EDI-3 weist Homologie zu einem funktionell bisher nicht charakterisierten Transkript von 5444 bp (Zugangsnummer AL109935.39.1.178601, SEQ ID NO:7) mit einem vorhergesagten offenen Leserahmen von 2016 bp auf. Gemäß Datenbankinformation besteht das entsprechende Gen aus 20 Exons und liegt auf dem kurzen Arm von Chromosom 20 in der Bande p13. Eine Northern Blot-Analyse bestätigte die erwartete Transkriptgröße (Abb. 1).

### Beispiel 3: Quantitative RT-PCR

Eine TaqMan-Analyse erfolgte wie kürzlich beschrieben (Mohrmann G et al., Int. J. Cancer, in press, 2005). Zusanunengefasst wurde Gesamt-RNA aus Tumorgewebe unter Verwendung des RNeasy Mini Kit (Qiagen, Hilden, Deutschland) isoliert und durch Messung der optischen Dichte bei 260 nm quantifiziert. 2 µg Gesamt-RNA wurden für einen cDNA-Synthese-Ansatz verwendet, der 2,5 µl MultiScribe-reverse Transkriptase (50 U/µl, Applied Biosystems), 10 µl RT-Puffer, 22 µl 25 mM MgCl₂, 20 µl dNTP-Mix (Applied Biosystems), 2 µl RNase-Inhibitor (20 U/µl, Applied Biosystems), 5 µl zufällige Hexamere (50 µM, Applied Biosystems) in einem Gesamtvolumen von 100 µl enthielt. Das Gemisch wurde 2 Minuten bei 25°C, 30 Minuten bei 48°C inkubiert und das Enzym 5 Minuten bei 95°C inaktiviert. Alle cDNAs wurden durch Zugabe von 150 µl DEPC-behandeltem Wasser verdünnt und bei - 20°C gelagert. Für eine quantitative PCR-Analyse wurde die Taq-Man™ -PCR-Technologie eingesetzt (Gene Amp 5700 Sequenz-Nachweissystem, ABI, Weiterstadt, Deutschland). Eine PCR erfolgte in einem Volumen von 25 µl mit 12,5 µl SYBR GREEN-PCR-Mastermix (einschließlich Enzympuffer, Fluoreszenzfarbstoff und Nukleotide, Applied Biosystems), 5 µM von jedem Primer (2,5 µl, 10 mM), 2,5 µl DEPC-behandeltem Wasser und 5 µl cDNA-Matrize. Zwei Primerpaare wurden für eine *EDI-3*-Analyse verwendet: (i) 5'-TTTCA AAATG CTGCA GGGTA AT-3' und 5'-ACCCA CAAAG CAACA GTGTG TA-3', (ii) 5'-CACAA TCTGC TTCTA ATCCA AGAA-3' und 5'-TGCTT TGTGG GTTTG TTTTG TA-3'. Die PCR umfasste ein Vorinkubation für 2 Minuten bei 50°C, gefolgt von einer Denaturierung für 10 Minuten bei 95°C. 40 Zyklen wurden durchgeführt, einschließlich einer Denaturierung für 15 Sekunden bei 95°C, einer Hybridisierung für 60 Sekunden bei 60°C und einer Verlängerung für 30 Sekunden bei 72°C. Die Reaktion wurde von dem Dissoziationsprotokoll gefolgt, wodurch der Bereich zwischen 60 und 94°C untersucht wurde. Emissionsbereiche des Fluoreszenzfarbstoffs wurden in Echtzeit während der PCR gemessen und die relative mRNA-Quantifizierungswerte wurden aus der Schwellenzykluszahl erhalten, aus der der Anstieg des Signals, das mit einem exponentiellen Wachstum von PCR-Produkt assoziiert ist, nachweisbar wurde. Die Sequenznachweissystem-Software, Version 1.6 (ABI, Weiterstadt, Deutschland) wurde eingesetzt. Die Quantifizierung wurde durch das konstitutive Gen huPO (menschliches Phosphoprotein) gemäß Vlachtsis et al. (Vlachtsis K et al., Oncol Rep. 9:1133-8, 2002) normalisiert. In alle PCR-Analysen wurde eine Negativkontrolle ohne reverse Transkriptase eingeschlossen.

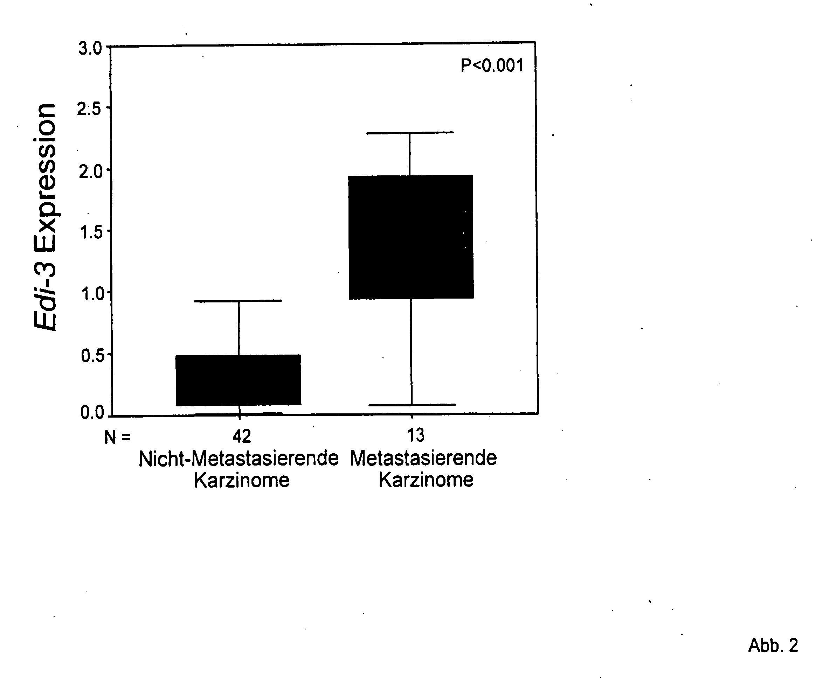

Unter Verwendung des "Validierungssatzes von Karzinomen" wurde untersucht, ob der Unterschied hinsichtlich der Expression von *EDI-3,* der in dem "Screeningsatz von Tumoren" festgestellt wurde, bestätigt werden konnte. Eine quantitative RT-PCR zeigte eine 6,4-fach höhere Expression von *EDI-3* in metastasierenden endometrialen Karzinomen im Vergleich zu nicht-metastasierenden endometrialen Karzinomen (p<0,001, Abb. 2). Ähnliche Ergebnisse wurden in einer unabhängigen Untersuchung der gleichen RNA-Spezies unter Verwendung eines zweiten Primerpaares erhalten (Abb. 3 und 4).

### Beispiel 4: Eine Expression von EDI-3 zeigt ein Rezidiv-freies Überleben an

Falls eine Expression von *EDI-3* mit einer Bildung von Metastasen assoziiert ist, könnte man annehmen, dass *EDI-3* ein Anzeichen für ein Ausbleiben eines Rezidivs ist. Tatsächlich war die Expression von *EDI-3* (dichotomisiert bei dem Percentil von 75%) mit einem Rezidiv-freien Überleben assoziiert (Figur 5). Die mittlere Zeitspanne bis zu einem Rezidiv betrug 1,47 Jahre im Fall von Patienten, die große Mengen an *EDI-3* exprimierten. Im Gegensatz dazu waren 79% der Patienten mit einer geringen Expression von *EDI-3* 5 Jahre nach einer Operation tumorfrei (p=0,0023). Unter Verwendung des proportionalen Hazard-Modells war *EDI-3* signifikant in einer univariaten Analyse (RR=4,3, P=0,002), sowie in einer multivariaten Step-Up-Regressionsanalyse mit dem FIGO-Stadium (I, II gegenüber III, IV), einem Alter über 70 Jahren, Diabetes mellitus (j/n), der Gradierung (1, 2 gegenüber 3) und der Tiefe einer Invasion in das Myometrium (0-2 mm gegenüber 3+ mm) als Covariaten (RR=3,6, P=0,012). Nur die Expression von *EDI-3* und das FIGO-Stadium waren in einer multivariaten Analyse prognostisch (Tabelle 2).

**Tabelle 2. Assoziierung der EDI-3-Expression mit tumorfreiem Überleben in 57 Patienten mit primärem Krebs des Endometriums ("Validierungssatz an Tumoren") unter Verwendung des univariaten und multivariaten proportionalen Hazard-Modelts (Cox-Analyse).**

| **Univariate Analyse** | | | |
|---|---|---|---|
| Faktor | Relatives Risiko | 95%-Konfidenzintervall | p-Wert |
| *EDI-3* mRNA | 4,3 | 1,7 - 11,0 | 0,002 |

| **Multivariate Analyse** | | | |
|---|---|---|---|
| Angepaßt an FIGO-Stadium (I, II vs. III, IV), Gradierung (1, 2 vs. 3), Invasionstiefe (0-2 mm vs 3+ mm), Alter (jünger gegenüber älter als 70 Jahre) und Diabetes mellitus | | | |
| Faktor | Relatives Risiko | 95%-Konfidenzintervall | p-Wert |
| *EDI-3* mRNA | 3,6 | 1,3 - 9,7 | 0,012 |
| FIGO-Stadium (Stadium I, II vs III, IV) | 5,1 | 1,8 - 14,0 | 0,002 |

Eine Anpassung bezüglich des FIGO-Stadiums könnte aufgrund einer möglichen Verletzung der proportionalen Hazard-Annahme, die dem Cox-Modell unterliegt, und der kleinen Probengröße problematisch sein. Jedoch bleibt die Wirkung von *EDI-3* signifikant, falls das FIGO-Stadium als ein Faktor in das Cox-Modell einbezogen wird (p=0,012), als auch wenn das FIGO-Stadium für eine Stratifizierung eingesetzt wird, wobei unterschiedliche Hazard-Funktionen für eine begrenzte (FIGO I, II) und fortgeschrittene (FIGO III, IV) Erkrankung angenommen werden (p<0,001, Abb. 6). Um die Reproduzierbarkeit der quantitativen RT-PCR zu überprüfen, wurden zusätzlich die gleichen mRNA-Spezies unter Verwendung eines zweiten Primerpaares, das gegen eine Region des *EDI-3-*Transkripts gerichtet ist, die weiter stromabwärts liegt, untersucht. Daten, die mit dem zweiten Primerpaar erhalten wurden, stimmten mit denjenigen des ersten Primerpaars überein (p<0,001, R=0,824) und bestätigten eine Verbindung zwischen der *EDI-3*-Expression und einem Rezidiv-freien Überleben (Tabelle 3, Abb. 7).

**Tabelle 3. Bestätigungsexperiment unter Verwendung eines zweiten Primerpaares für eine Quantifizierung der EDI-3-mRNA-Expression. Ähnlich zu den Ergebnissen, die mit dem ersten Primerpaar erhalten wurden, war eine Expression des EDI-3-Transkripts mit tumorfreiem Überleben in 57 Patienten mit primärem Krebs des Endometriums ("Validierungssatz an Tumoren") bei Verwendung des univariaten und multivariaten proportionalen Hazard-Modells (Cox-Analyse) assoziiert.**

| **Univariate Analyse** | | | |
|---|---|---|---|
| Faktor | Relatives Risiko | 95%-Konfidenzintervall | p-Wert |
| *EDI-3* mRNA | 7,9 | 3,0 - 21,3 | <0,001 |

| **Multivariate Analyse** | | | |
|---|---|---|---|
| Angepaßt an FIGO-Stadium (I, II vs. III, IV), Gradierung (1, 2 vs. 3), Invasionstiefe (0-2 mm vs 3+ mm), Alter (jünger gegenüber älter als 70 Jahre) und Diabetes mellitus | | | |
| Faktor | Relatives Risiko | 95%-Konfidenzintervall | p-Wert |
| *EDI-3* mRNA | 7,3 | 2,7 - 19,9 | <0,001 |

## Patentansprüche

1. Verfahren zur Diagnose und/oder Überwachung eines endometrialen Tumors bei einem Patienten, umfassend
(i) den Nachweis und/oder die Bestimmung der Menge einer Nukleinsäure, die ausgewählt ist aus der Gruppe bestehend aus:
(a) einer Nukleinsäure, die eine Nukleinsäuresequenz umfasst, die aus der Gruppe bestehend aus SEQ ID NO: 3, 6 und 7 und einem Teil von mindestens 30 aufeinanderfolgenden Nukleotiden davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist, und
(d) einer Nukleinsäure, die zu der Nukleinsäure unter (a), (b) oder (c) komplementär ist, und/oder
(ii) den Nachweis und/oder die Bestimmung der Menge eines durch die Nukleinsäure unter (i) kodierten Proteins oder Peptids oder eines Teils davon,
in einer aus einem Patienten isolierten biologischen Probe, die endometriales Körpergewebe umfasst,
wobei ein Vorliegen der Nukleinsäure, des Proteins oder Peptids oder des Teils davon und/oder eine im Vergleich zu einem Patienten ohne einen endometrialen Tumor, ohne ein Risiko für einen endometrialen Tumor, ohne eine Metastasierung eines endometrialen Tumors und/oder ohne ein Risiko für eine Metastasierung eines endometrialen Tumors erhöhte Menge der Nukleinsäure, des Proteins oder Peptids oder des Teils davon auf das Vorliegen eines endometrialen Tumors, ein Risiko für einen endometrialen Tumor, eine Metastasierung eines endometrialen Tumors und/oder ein Risiko für eine Metastasierung eines endometrialen Tumors hinweist.

2. Verfahren zur Bewertung und/oder Prognostizierung des Metastasierungsverhaltens und/oder des Auftretens eines Rezidivs eines endometrialen Tumors, umfassend
(i) den Nachweis und/oder die Bestimmung der Menge einer Nukleinsäure, die ausgewählt ist aus der Gruppe bestehend aus:
(a) einer Nukleinsäure, die eine Nukleinsäuresequenz umfasst, die aus der Gruppe bestehend aus SEQ ID NO: 3, 6 und 7 und einem Teil von mindestens 30 aufeinanderfolgenden Nukleotiden davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist, und
(d) einer Nukleinsäure, die zu der Nukleinsäure unter (a), (b) oder (c) komplementär ist, und/oder
(ii) den Nachweis und/oder die Bestimmung der Menge eines durch die Nukleinsäure unter (i) kodierten Proteins oder Peptids oder eines Teils davon,
in einer aus einem Patienten isolierten biologischen Probe, die endometriales Körpergewebe umfasst,
wobei ein Vorliegen der Nukleinsäure, des Proteins oder Peptids oder des Teils davon und/oder eine im Vergleich zu einem Patienten ohne einen endometrialen Tumor, ohne ein Risiko für einen endometrialen Tumor, ohne eine Metastasierung eines endometrialen Tumors, ohne ein Risiko für eine Metastasierung eines endometrialen Tumors, ohne ein Rezidiv eines endometrialen Tumors und/oder ohne ein Risiko für ein Rezidiv eines endometrialen Tumors erhöhte Menge der Nukleinsäure, des Proteins oder Peptids oder des Teils davon auf das Vorliegen einer Metastasierung oder eines Rezidivs eines endometrialen Tumors oder ein Risiko für eine Metastasierung oder ein Rezidiv eines endometrialen Tumors hinweist.

3. Verfahren nach einem der Ansprüche 1-2, das eine Unterscheidung zwischen benignen und malignen Veränderungen ermöglicht.

4. Verfahren nach einem der Ansprüche 1-3, wobei der Nachweis und/oder die Bestimmung der Menge
(i) die Kontaktierung der biologischen Probe mit einem Mittel, das spezifisch an die Nukleinsäure oder an das Protein oder Peptid oder den Teil davon bindet, und
(ii) den Nachweis der Komplexbildung zwischen dem Mittel und der Nukleinsäure oder dem Protein oder Peptid oder dem Teil davon umfasst.

5. Verfahren nach Anspruch 4, wobei
(i) das Mittel, das spezifisch an die Nukleinsäure bindet, ein Oligonukleotid oder Polynukleotid ist, das spezifisch mit der Nukleinsäure hybridisiert, oder
(ii) das Mittel, das spezifisch an das Protein oder Peptid oder den Teil davon bindet, ein Antikörper ist, der spezifisch an das Protein oder Peptid oder den Teil davon bindet.

6. Verfahren nach einem der Ansprüche 1 und 3-5, wobei die Überwachung eines endometrialen Tumors eine Bestimmung der Regression, des Verlaufs oder des Ausbruchs des endometrialen Tumors in einer Probe aus dem Patienten umfasst.

7. Verfahren nach einem der Ansprüche 1-6, wobei der endometriale Tumor ein metastasierender endometrialer Tumor ist.

## Claims

1. A method of diagnosing and/or monitoring an endometrial tumor in a patient, comprising
(i) detecting and/or determining the amount of a nucleic acid selected from the group consisting of:
(a) a nucleic acid which comprises a nucleic acid sequence selected from the group consisting of SEQ ID NO: 3, 6 and 7, and a part of at least 30 consecutive nucleotides thereof,
(b) a nucleic acid which hybridizes with the nucleic acid of (a) under stringent conditions,
(c) a nucleic acid which is degenerated with respect to the nucleic acid of (a) or (b), and
(d) a nucleic acid which is complementary to the nucleic acid of (a), (b) or (c), and/or
(ii) detecting and/or determining the amount of a protein or peptide encoded by the nucleic acid of (i) or of a part thereof,
in a biological sample comprising endometrial body tissue isolated from a patient,
wherein a presence of the nucleic acid, the protein or peptide or the part thereof and/or an increased amount of said nucleic acid, said protein or peptide or said part thereof in comparison with a patient without an endometrial tumor, without a risk of an endometrial tumor, without metastasis of an endometrial tumor and/or without a risk of metastasis of an endometrial tumor indicates the presence of an endometrial tumor, a risk of an endometrial tumor, metastasis of an endometrial tumor and/or a risk of metastasis of an endometrial tumor.

2. A method of evaluating and/or predicting the metastatic behavior and/or the recurrence of an endometrial tumor, comprising
(i) detecting and/or determining the amount of a nucleic acid selected from the group consisting of:
(a) a nucleic acid which comprises a nucleic acid sequence selected from the group consisting of SEQ ID NO: 3, 6 and 7 and a part of at least 30 consecutive nucleotides thereof,
(b) a nucleic acid which hybridizes with the nucleic acid of (a) under stringent conditions,
(c) a nucleic acid which is degenerated with respect to the nucleic acid of (a) or (b), and
(d) a nucleic acid which is complementary to the nucleic acid of (a), (b) or (c), and/or
(ii) detecting and/or determining the amount of a protein or peptide encoded by the nucleic acid of (i) or of a part thereof,
in a biological sample comprising endometrial body tissue isolated from a patient,
wherein a presence of the nucleic acid, the protein or peptide or the part thereof and/or an increased amount of said nucleic acid, said protein or peptide or said part thereof in comparison with a patient without an endometrial tumor, without a risk of an endometrial tumor, without metastasis of an endometrial tumor, without a risk of metastasis of an endometrial tumor, without a recurrence of an endometrial tumor and/or without a risk of a recurrence of an endometrial tumor indicates the presence of metastasis or recurrence of an endometrial tumor or a risk of metastasis or recurrence of an endometrial tumor.

3. The method as claimed in any of claims 1-2, which enables benign and malignant changes to be distinguished.

4. The method as claimed in any of claims 1-3, wherein detection and/or determination of the amount comprises
(i) contacting the biological sample with an agent which binds specifically to the nucleic acid or to the protein or peptide or the part thereof, and
(ii) detecting the formation of a complex between said agent and said nucleic acid or said protein or peptide or said part thereof.

5. The method as claimed in claim 4, wherein
(i) the agent which binds specifically to the nucleic acid is an oligonucleotide or polynucleotide which specifically hybridizes with said nucleic acid, or
(ii) the agent which binds specifically to the protein or peptide or the part thereof is an antibody which binds specifically to said protein or peptide or said part thereof.

6. The method as claimed in any of claims 1 and 3-5, wherein monitoring an endometrial tumor comprises determining the regression, the course or the onset of said endometrial tumor in a sample from the patient.

7. The method as claimed in any of claims 1-6, wherein the endometrial tumor is a metastasizing endometrial tumor.

## Revendications

1. Procédé pour le diagnostic et/ou la prévention d'une tumeur endométriale chez un patient, comprenant
(i) la caractérisation et/ou la détermination de la quantité d'un acide nucléique qui est choisi dans le groupe consistant en:
(a) un acide nucléique, qui comporte une séquence d'acide nucléique qui est choisie dans le groupe consistant en SEQ ID NO: 3, 6 et 7 et une portion d'au moins 30 nucléotides consécutifs de celle-ci,
(b) un acide nucléique qui hybride dans des conditions stringentes avec l'acide nucléique selon (a),
(c) un acide nucléique qui est dégénéré en ce qui concerne l'acide nucléique sous (a) ou (b), et
(d) un acide nucléique qui est complémentaire de l'acide nucléique sous (a), (b) ou (c), et/ou
(ii) la caractérisation et/ou la détermination de la quantité d'une protéine ou d'un peptide ou d'une portion de ceux-ci codé au moyen de l'acide nucléique sous (i),
dans un échantillon biologique isolé à partir d'un patient, qui comporte des tissus corporels endométriaux,
tandis qu'une présence de l'acide nucléique, de la protéine ou du peptide ou de la portion de ceux-ci et/ou que, par comparaison avec un patient sans tumeur endométriale, sans risque de tumeur endométriale, sans développement de métastases d'une tumeur endométriale et/ou sans risque de développement de métastases d'une tumeur endométriale, une quantité accrue de l'acide nucléique, de la protéine ou du peptide ou de la portion de ceux-ci indique la présence d'une tumeur endométriale, d'un risque de tumeur endométriale, d'un développement de métastases d'une tumeur endométriale et/ou d'un risque de développement de métastases d'une tumeur endométriale.

2. Procédé pour l'évaluation et/ou le pronostic du comportement vis-à-vis du développement de métastases et/ou de l'apparition d'une récidive de tumeur endométriale, comprenant
(i) la caractérisation et/ou la détermination de la quantité d'un acide nucléique qui est choisi dans le groupe consistant en:
(a) un acide nucléique, qui comporte une séquence d'acide nucléique qui est choisie dans le groupe consistant en SEQ ID NO: 3, 6 et 7 et une portion d'au moins 30 nucléotides consécutifs de celle-ci,
(b) un acide nucléique qui hybride dans des conditions stringentes avec l'acide nucléique selon (a),
(c) un acide nucléique qui est dégénéré en ce qui concerne l'acide nucléique sous (a) ou (b), et
(d) un acide nucléique qui est complémentaire de l'acide nucléique sous (a), (b) ou (c), et/ ou
(ii) la caractérisation et/ou la détermination de la quantité d'une protéine ou d'un peptide ou d'une portion de ceux-ci codé au moyen de l'acide nucléique sous (i),
dans un échantillon biologique isolé à partir d'un patient, qui comporte des tissus corporels endométriaux,
tandis qu'une présence de l'acide nucléique, de la protéine ou du peptide ou de la portion de ceux-ci et/ ou que, par comparaison avec un patient sans tumeur endométriale, sans risque de tumeur endométriale, sans développement de métastases d'une tumeur endométriale, sans risque de développement de métastases d'une tumeur endométriale, sans risque de tumeur endométriale et/ ou sans risque de récidive de tumeur endométriale, une quantité accrue de l'acide nucléique, de la protéine ou du peptide ou de la portion de ceux-ci indique la présence d'un développement de métastases ou d'une récidive de tumeur endométriale, ou un risque de développement de métastases ou d'une récidive de tumeur endométriale.

3. Procédé selon l'une des revendications 1-2, qui permet une distinction entre des altérations bénignes et malignes.

4. Procédé selon l'une des revendications 1-3, dans lequel la caractérisation et/ ou la détermination de la quantité comprend
(i) la mise en contact de l'échantillon biologique avec un moyen qui se lie spécifiquement à l'acide nucléique ou à la protéine ou au peptide ou à la portion de ceux-ci, et
(ii) la caractérisation de la formation d'un complexe entre le moyen et l'acide nucléique ou la protéine ou le peptide ou la portion de ceux-ci.

5. Procédé selon la revendication 4, dans lequel
(i) le moyen qui se lie spécifiquement à l'acide nucléique est un oligonucléotide ou un polynucléotide qui hybride spécifiquement avec l'acide nucléique, ou
(ii) le moyen qui se lie spécifiquement à la protéine ou au peptide ou à la portion de ceux-ci est un anticorps qui se lie spécifiquement à la protéine ou au peptide ou à la portion de ceux-ci.

6. Procédé selon l'une des revendications 1 et 3-5, dans lequel la prévention d'une tumeur endométriale comporte une détermination de la régression, de l'évolution ou de la disparition de la tumeur endométriale dans un échantillon provenant du patient.

7. Procédé selon l'une des revendications 1-6, dans lequel la tumeur endométriale est une tumeur endométriale en cours de métastase.


### SEQUENZPROTOKOLL

<110> Johannes Gutenberg-Universität Mainz, vertreten durch den Präsidenten, et al.
<120> Molekulare Marker für eine Tumordiagnose und -therapie
<130> 410-3 PCT
<150> DE 10 2005 059 242.2
   <151> 2005-12-12
<160> 31
<170> PatentIn version 3.1
<210> 1
   <211> 162
   <212> DNA
   <213> Homo sapiens
<220>
   <221> misc_feature
   <222> (5)..(5)
   <223> unsure
<220>
   <221> misc_feature
   <222> (8)..(8)
   <223> unsure
<220>
   <221> misc_feature
   <222> (17)..(17)
   <223> unsure
<220>
   <221> misc_feature
   <222> (20)..(20)
   <223> unsure
<220>
   <221> misc_feature
   <222> (25)..(25)
   <223> unsure
<220>
   <221> misc_feature
   <222> (26)..(26)
   <223> unsure
<220>
   <221> misc_feature
   <222> (29)..(29)
   <223> unsure
<220>
   <221> misc_feature
   <222> (36)..(36)
   <223> unsure
<220>
   <221> misc_feature
   <222> (44)..(44)
   <223> unsure
<220>
   <221> misc_feature
   <222> (46)..(46)
   <223> unsure
<220>
   <221> misc_feature
   <222> (50)..(50)
   <223> unsure
<400> 1
   ttaanccnct ttttatnccn ttaannaana aaatantttc agtncntcan gagctgggaa 60
   actgtatcca ggatgtactc aaacacatgt ttacatattg tctaccgagc ttcaggctga 120
   attagtaata gcttactgcc aaaaggataa tatttatgac ca 162
<210> 2
   <211> 51
   <212> DNA
   <213> Homo sapiens
<220>
   <221> misc_feature
   <222> (30)..(30)
   <223> unsure
<400> 2
   cccttctccc tgcactcaat aaaccctcan taaatattct cattgtcaat c 51
<210> 3
   <211> 156
   <212> DNA
   <213> Homo sapiens
<220>
   <221> misc_feature
   <222> (5)..(5)
   <223> unsure
<400> 3
   tgcanactta gtagaataga tcacaacata caaattcaat tcagtgcatg ctttaggtgt 60
   taagcatgag attgtacatg tttactgtta ggtccttgca tctgtggtgc taggtgagta 120
   tgagaagatg tcaaggactg gacgtatttt tgttgc 156
<210> 4
   <211> 126
   <212> DNA
   <213> Homo sapiens
<220>
   <221> misc_feature
   <222> (8)..(8)
   <223> unsure
<220>
   <221> misc_featüre
   <222> (10)..(10)
   <223> unsure
<220>
   <221> misc_feature
   <222> (14)..(14)
   <223> unsure
<400> 4
   tttcagtncn tcangagctg ggaaactgta tccaggatgt actcaaacac atgtttacat     60
   attgtctacc gagcttcagg ctgaattagt aatagcttac tgccaaaagg ataatattta    120
   tgacca                                                               126
<210> 5
   <211> 70
   <212> DNA
   <213> Homo sapiens
<400> 5
   acatattgtc taccgagctt caggctgaat tagtaatagc ttactgccaa aaggataata     60
   tttatgacca                                                            70
<210> 6
   <211> 145
   <212> DNA
   <213> Homo sapiens
<400> 6
   acttagtaga atagatcaca acatacaaat tcaattcagt gcatgcttta ggtgttaagc     60
   atgagattgt acatgtttac tgttaggtcc ttgcatctgt ggtgctaggt gagtatgaga    120
   agatgtcaag gactggacgt atttt                                          145
<210> 7
   <211> 5444
   <212> DNA
   <213> Homo sapiens
<220>
   <221> CDS
   <222> (206)..(2224)
   <223>
<400> 7
   ctggagcagc tgaaaccggt ttgagcgtgg ctgcttcctg ccgctcgacg ccgcggcagg     60
   ccgcctgggg ggagcgctgg cgaggcacgg acggcgggcg cccggtacct ctgcccgcgg    120
   tcctcgctct cgggcggggc ggcggcgacg cggacctgcg gactagcgaa cccggagcac    180
   gacatcataa aataaatcca tcaga atg aca cct tct cag gtt gcc ttt gaa      232
                               Met Thr Pro Ser Gln Val Ala Phe Glu
                               1               5
   ata aga gga act ctt tta cca gga gaa gtt ttt gcg ata tgt gga agc    280
   Ile Arg Gly Thr Leu Leu Pro Gly Glu Val Phe Ala Ile Cys Gly Ser
   10                  15                  20                  25
   tgt gat gct ttg gga aac tgg aat cct caa aat gct gtg gct ctt ctt    328
   Cys Asp Ala Leu Gly Asn Trp Asn Pro Gln Asn Ala Val Ala Leu Leu
                   30                  35                  40
   cca gag aat gac aca ggt gaa agc atg cta tgg aaa gca acc att gta    376
   Pro Glu Asn Asp Thr Gly Glu Ser Met Leu Trp Lys Ala Thr Ile Val
               45                  50                  55
   ctc agt aga gga gta tca gtt cag tat cgc tac ttc aaa ggg tac ttt    424
   Leu Ser Arg Gly Val Ser Val Gln Tyr Arg Tyr Phe Lys Gly Tyr Phe
           60                  65                  70
   tta gaa cca aag act atc ggt ggt cca tgt caa gtg ata gtt cac aag    472
   Leu Glu Pro Lys Thr Ile Gly Gly Pro Cys Gln Val Ile Val His Lys
       75                  80                  85
   tgg gag act cat cta caa cca cga tca ata acc cct tta gaa agc gaa    520
   Trp Glu Thr His Leu Gln Pro Arg Ser Ile Thr Pro Leu Glu Ser Glu
   90                  95                  100                 105
   att att att gac gat gga caa ttt gga atc cac aat ggt gtt gaa act    568
   Ile Ile Ile Asp Asp Gly Gln Phe Gly Ile His Asn Gly Val Glu Thr
                   110                 115                 120
   ctg gat tct gga tgg ctg aca tgt cag act gaa ata aga tta cgt ttg    616
   Leu Asp Ser Gly Trp Leu Thr Cys Gln Thr Glu Ile Arg Leu Arg Leu
               125                 130                 135
   cat tat tct gaa aaa cct cct gtg tca ata acc aag aaa aaa tta aaa    664
   His Tyr Ser Glu Lys Pro Pro Val Ser Ile Thr Lys Lys Lys Leu Lys
           140                 145                 150
   aaa tct aga ttt agg gtg aag ctg aca cta gaa ggc ctg gag gaa gat    712
   Lys Ser Arg Phe Arg Val Lys Leu Thr Leu Glu Gly Leu Glu Glu Asp
       155                 160                 165
   gac gat gat agg gta tct ccc act gta ctc cac aaa atg tcc aat agc    760
   Asp Asp Asp Arg Val Ser Pro Thr Val Leu His Lys Met Ser Asn Ser
   170                 175                 180                 185
   ttg gag ata tcc tta ata agc gac aat gag ttc aag tgc agg cat tca    808
   Leu Glu Ile Ser Leu Ile Ser Asp Asn Glu Phe Lys Cys Arg His Ser
                   190                 195                 200
   cag ccg gag tgt ggt tat ggc ttg cag cct gat cgt tgg aca gag tac    856
   Gln Pro Glu Cys Gly Tyr Gly Leu Gln Pro Asp Arg Trp Thr Glu Tyr
               205                 210                 215
   agc ata cag acg atg gaa cca gat aac ctg gaa cta atc ttt gat ttt    904
   Ser Ile Gln Thr Met Glu Pro Asp Asn Leu Glu Leu Ile Phe Asp Phe
           220                 225                 230
   ttc gaa gaa gat ctc agt gag cac gta gtt cag ggt gat gcc ctt cct    952
   Phe Glu Glu Asp Leu Ser Glu His Val Val Gln Gly Asp Ala Leu Pro
       235                 240                 245
   gga cat gtg ggt aca gct tgt ctc tta tca tcc acc att gct gag agt    1000
   Gly His Val Gly Thr Ala Cys Leu Leu Ser Ser Thr Ile Ala Glu Ser
   250                 255                 260                 265
   gga aag agt gct gga att ctt act ctt ccc atc atg agc aga aat tcc    1048
   Gly Lys Ser Ala Gly Ile Leu Thr Leu Pro Ile Met Ser Arg Asn Ser
                   270                 275                 280
   cgg aaa aca ata ggc aaa gtg aga gtt gac tat ata att att aag cca      1096
   Arg Lys Thr Ile Gly Lys Val Arg Val Asp Tyr Ile Ile Ile Lys Pro
               285                 290                 295
   tta cca gga tac agt tgt gac atg aaa tct tca ttt tcc aag tat tgg      1144
   Leu Pro Gly Tyr Ser Cys Asp Met Lys Ser Ser Phe Ser Lys Tyr Trp
           300                 305                 310
   aag cca aga ata cca ttg gat gtt ggc cat cga ggt gca gga aac tct      1192
   Lys Pro Arg Ile Pro Leu Asp Val Gly His Arg Gly Ala Gly Asn Ser
       315                 320                 325
   aca aca act gcc cag ctg gct aaa gtt caa gaa aat act att gct tct      1240
   Thr Thr Thr Ala Gln Leu Ala Lys Val Gln Glu Asn Thr Ile Ala Ser
   330                 335                 340                 345
   tta aga aat gct gct agt cat ggt gca gcc ttt gta gaa ttt gac gta      1288
   Leu Arg Asn Ala Ala Ser His Gly Ala Ala Phe Val Glu Phe Asp Val
                   350                 355                 360
   cac ctt tca aag gac ttt gtg ccc gtg gta tat cat gat ctt acc tgt      1336
   His Leu Ser Lys Asp Phe Val Pro Val Val Tyr His Asp Leu Thr Cys
               365                 370                 375
   tgt ttg act atg aaa aag aaa ttt gat gct gat cca gtt gaa tta ttt      1384
   Cys Leu Thr Met Lys Lys Lys Phe Asp Ala Asp Pro Val Glu Leu Phe
           380                 385                 390
   gaa att cca gta aaa gaa tta aca ttt gac caa ctc cag ttg tta aag      1432
   Glu Ile Pro Val Lys Glu Leu Thr Phe Asp Gln Leu Gln Leu Leu Lys
       395                 400                 405
   ctc act cat gtg act gca ctg aaa tct aag gat cgg aaa gaa tct gtg      1480
   Leu Thr His Val Thr Ala Leu Lys Ser Lys Asp Arg Lys Glu Ser Val
   410                 415                 420                 425
   gtt cag gag gaa aat tcc ttt tca gaa aat cag cca ttt cct tct ctt      1528
   Val Gln Glu Glu Asn Ser Phe Ser Glu Asn Gln Pro Phe Pro Ser Leu
                   430                 435                 440
   aag atg gtt tta gag tct ttg cca gaa gat gta ggg ttt aac att gaa      1576
   Lys Met Val Leu Glu Ser Leu Pro Glu Asp Val Gly Phe Asn Ile Glu
               445                 450                 455
   ata aaa tgg atc tgc cag caa agg gat gga atg tgg gat ggt aac tta      1624
   Ile Lys Trp Ile Cys Gln Gln Arg Asp Gly Met Trp Asp Gly Asn Leu
           460                 465                 470
   tca aca tat ttt gac atg aat ctg ttt ttg gat ata att tta aaa act      1672
   Ser Thr Tyr Phe Asp Met Asn Leu Phe Leu Asp Ile Ile Leu Lys Thr
       475                 480                 485
   gtt tta gaa aat tct ggg aag agg aga ata gtg ttt tct tca ttt gat      1720
   Val Leu Glu Asn Ser Gly Lys Arg Arg Ile Val Phe Ser Ser Phe Asp
   490                 495                 500                 505
   gca gat att tgc aca atg gtt cgg caa aag cag aac aaa tat ccg ata      1768
   Ala Asp Ile Cys Thr Met Val Arg Gln Lys Gln Asn Lys Tyr Pro Ile
                   510                 515                 520
   cta ttt tta act caa gga aaa tct gag att tat cct gaa ctc atg gac      1816
   Leu Phe Leu Thr Gln Gly Lys Ser Glu Ile Tyr Pro Glu Leu Met Asp
               525                 530                 535
   ctc aga tct cgg aca acc ccc att gca atg agc ttt gca cag ttt gaa      1864
   Leu Arg Ser Arg Thr Thr Pro Ile Ala Met Ser Phe Ala Gln Phe Glu
           540                 545                 550
   aat cta ctg ggg ata aat gta cat act gaa gac ttg ctc aga aac cca      1912
   Asn Leu Leu Gly Ile Asn Val His Thr Glu Asp Leu Leu Arg Asn Pro
       555                 560                 565
   tcc tat att caa gag gca aaa gct aag gga cta gtc ata ttc tgc tgg      1960
   Ser Tyr Ile Gln Glu Ala Lys Ala Lys Gly Leu Val Ile Phe Cys Trp
   570                 575                 580                 585
   ggt gat gat acc aat gat cct gaa aac aga agg aaa ttg aag gaa ctt      2008
   Gly Asp Asp Thr Asn Asp Pro Glu Asn Arg Arg Lys Leu Lys Glu Leu
                   590                 595                 600
   gga gtt aat ggt cta att tat gat agg ata tat gat tgg atg cct gaa      2056
   Gly Val Asn Gly Leu Ile Tyr Asp Arg Ile Tyr Asp Trp Met Pro Glu
               605                 610                 615
   caa cca aat ata ttc caa gtg gag caa ttg gaa cgc ctg aag cag gaa      2104
   Gln Pro Asn Ile Phe Gln Val Glu Gln Leu Glu Arg Leu Lys Gln Glu
           620                 625                 630
   ttg cca gag ctt aag agc tgt ttg tgt ccc act gtt agc cgc ttt gtt      2152
   Leu Pro Glu Leu Lys Ser Cys Leu Cys Pro Thr Val Ser Arg Phe Val
       635                 640                 645
   ccc tca tct ttg tgt ggg gag tct gat atc cat gtg gat gcc aac ggc      2200
   Pro Ser Ser Leu Cys Gly Glu Ser Asp Ile His Val Asp Ala Asn Gly
   650                 655                 660                 665
   att gat aac gtg gag aat gct tag tttttattgc acagaggtca ttttgggggc    2254
   Ile Asp Asn Val Glu Asn Ala
                   670
   gtgcaccgct gttctgggta ttcatttttc atcactgagc attgttgatc tatgcctttt    2314
   gggcttctca gttcaatgaa gcaataatga agtatttaac tctttcacta cagttcttgc    2374
   aagtatgcta tttaaattac ttggccaggt ataattgcca gtcagtctct ttatagtgag    2434
   aaaatttatt ggttagtaat ataaatattt taaactaaat atataaatct ataatgttaa    2494
   acatatgttc attaaaagca tagcactttg aaattaacta tataaatagc tcatatttac    2554
   acttacagct tttcatttga tcaggtctga aatctttagc acttaaggaa aatgactatg    2614
   cataattata cctgaccatg aaaaaaataa gtacctcaaa tgcatgcatt tgcactggtg    2674
   attccaactg cacaaatctt tgtgccatct tgtatatagg tattttttac atgggttgac    2734
   atgcacacaa caccattttc attcagtatg aaccttgagg ctgctgccat ttttccactt     2794
   aaccaaacca gcctgaaggt gaacctcgaa acttgtttca taaatctttc aaaagttgtt    2854
   ttacatcaat gttaaaattt caaaatgctg cagggtaatt taatgtataa aatattagta    2914
   agaaaaagta tgtattgcat acttagtaga atagatcaca acatacaaat tcaattcagt    2974
   gcatgcttta ggtgttaagc atgagattgt acatgtttac tgttaggtcc ttgcatctgt    3034
   ggtgctaggt gagtatgaga agatgtcaag gactggacgt attttgttgc ctaaaaaaaa    3094
   aaggctgttt gtaggcgttt taaatatgct tattttgtgt gtctctcact acctattaca    3154
   cactgttgct ttgtgggttt gttttgtatg tgcgtgtgtt atacagtagt taaatttcca    3214
   tgcagaaaaa taaatgtcct gaattttcat attagtattc tttattgtat atcatgcatg    3274
   taatttattt agaaatgtag gtcttactaa atgtatatgc atgtatttca gattatacta    3334
   ggatttcttg gattagaagc agattgtgtt aactgtaact taaagaatga atgttaaata 3394
   aaatgataca gatttatttt cttcattaca aaatgaaatt tcaagaaggt gttacttttg 3454
   tagaatggtt ttataatatg acaagaaatt ttaatatagt gtctacccta aagggatggc 3514
   ttatttgcat ctacctttta ctgcatgttt ttcacaaggc agtttattca tatattgaca 3574
   tattttggta gtagctgaga acctaagact tgaaattata cattgtgtag tattttttaa 3634
   gctaagcaat gcaattttgg tcagatctta tttgtgtgaa gataggctct gaaatcctat 3694
   ggtattgcgt ttgtaacgtt gatattaatg caaaatagtt taggaaatgg agtcttctgc 3754
   aagggttctg tatacttttc ccacattgta tgagattttc caaaattttg gtgtgaattg 3814
   ggcacttttg gaaaactcct gaaaaagaat tagtttcctt catctgcaga cctttgtcca 3874
   atacggttac catttcttta tagtaactcg attagccata tatgtttgtt tctagtcctg 3934
   ctcctttgct cctctcctat gccttcccag tgctggctcc attttgaaga ctcaaggaca 3994
   gaggggaagc agatcataaa gagaaaaagg agacagaaga aaggatgaag gaaggaggtc 4054
   atggggagtg tggcttctga gcagtttagt tgctggggag agcagacagt cactgcctac 4114
   aatacagaca gaaccttcct gctcactttc tgtcctatct cttcctgacc ttatgaacca 4174
   gtgttagtag atgattaaaa catgacaagc aatggctcct tattttcaca ggactaagtc 4234
   cgggccttcg tatcactagc tgttgccttt tacaccctgc ttcagccacc ctgtccctgt 4294
   cattggccct ggacttcctc tctgtgcccg tgtgtcctct gcctgggagc cctctcctcc 4354
   catagtcact ttctctctgc caaactcatt tcttcttgtg cccaagacct ctctcctgag 4414
   cccttgtgga aacttcagga aggatgaatc cgtctttgtg ctccacggct cgtaccttga 4474
   tcaggctgtg catcacagta attctgttct aggtaggcag agttgatctt tgtctcatct 4534
   gccaggctgc aggctcttca agggcaggga ccttgtcata gtcattttta ttttcacagt 4594
   gcttggaaca tggtggaaaa tgaatgttgg aattattgga gtaatataat ttgtatcaaa 4654
   tgtccttttg aattaagaga tttagttatg tttactaaga atgtaaactt tgaattggtt 4714
   tgcattttaa caattaggat ggtttattga tgtgaatttt gaaatgtaga ggtataatgt 4774
   taaattattt tatactttat ggaaatcaag tgaaatgttt gaaaaaatgc cgccattatc 4834
   ctctggtatt ttctactctc tggaattatg tgctgtaaat gatcggctgt aaatgtgagg 4894
   cacaccaccc acccctgtgt ggaaagtgtt gtggcgcttc ctgccaccca cccacctctc 4954
   tgccgttgct ccttgtgaca cttgtctgtc gtctcccatc caaactccaa gcttacagct 5014
   acctcagtac tgctttgctt gtctgaaaca cctcctttgc cttccttcag tgtcccgctc 5074
   aggtgcagcc tcctccctaa agctcatctc agcttttgat ctgaatgatg atggaaacat 5134
   gcagacagcc tctcagtctt actatttaat gttgtagctg ggaaaaaacc cagagaggtt 5194
   aactgatata ctgggttggg actaggatgt gggttttgtg actctgaatc ccatgttctc 5254
   aaactacgct gccttccgaa gtctggcatt tgttagctca tgcttccttg tagtccagct 5314
   tcttatgtgc ctgttatatt ctccagtaag attgtaagcc ccttaagggc agggacgtct 5374
   ttgcatctct agcactgcta tagtgttcta tccttagtta tgaactagat aaataaatgg 5434
   tggtggcaac 5444
<210> 8
   <211> 672
   <212> PRT
   <213> Homo sapiens
<400> 8
   Met Thr Pro Ser Gln Val Ala Phe Glu Ile Arg Gly Thr Leu Leu Pro
   1               5                   10                  15
   Gly Glu Val Phe Ala Ile Cys Gly Ser Cys Asp Ala Leu Gly Asn Trp
               20                  25                  30
   Asn Pro Gln Asn Ala Val Ala Leu Leu Pro Glu Asn Asp Thr Gly Glu
           35                  40                  45
   Ser Met Leu Trp Lys Ala Thr Ile Val Leu Ser Arg Gly Val Ser Val
       50                  55                  60
   Gln Tyr Arg Tyr Phe Lys Gly Tyr Phe Leu Glu Pro Lys Thr Ile Gly
   65                  70                  75                  80
   Gly Pro Cys Gln Val Ile Val His Lys Trp Glu Thr His Leu Gln Pro
                   85                  90                  95
   Arg Ser Ile Thr Pro Leu Glu Ser Glu Ile Ile Ile Asp Asp Gly Gln
               100                 105                 110
   Phe Gly Ile His Asn Gly Val Glu Thr Leu Asp Ser Gly Trp Leu Thr
           115                 120                 125
   Cys Gln Thr Glu Ile Arg Leu Arg Leu His Tyr Ser Glu Lys Pro Pro
       130                 135                 140
   Val Ser Ile Thr Lys Lys Lys Leu Lys Lys Ser Arg Phe Arg Val Lys
   145                 150                 155                 160
   Leu Thr Leu Glu Gly Leu Glu Glu Asp Asp Asp Asp Arg Val Ser Pro
                   165                 170                 175
   Thr Val Leu His Lys Met Ser Asn Ser Leu Glu Ile Ser Leu Ile Ser
               180                 185                 190
   Asp Asn Glu Phe Lys Cys Arg His Ser Gln Pro Glu Cys Gly Tyr Gly
           195                 200                 205
   Leu Gln Pro Asp Arg Trp Thr Glu Tyr Ser Ile Gln Thr Met Glu Pro
       210                 215                 220
   Asp Asn Leu Glu Leu Ile Phe Asp Phe Phe Glu Glu Asp Leu Ser Glu
   225 230 235 240
   His Val Val Gln Gly Asp Ala Leu Pro Gly His Val Gly Thr Ala Cys
   245 250 255
   Leu Leu Ser Ser Thr Ile Ala Glu Ser Gly Lys Ser Ala Gly Ile Leu
   260 265 270
   Thr Leu Pro Ile Met Ser Arg Asn Ser Arg Lys Thr Ile Gly Lys Val
   275 280 285
   Arg Val Asp Tyr Ile Ile Ile Lys Pro Leu Pro Gly Tyr Ser Cys Asp
   290 295 300
   Met Lys Ser Ser Phe Ser Lys Tyr Trp Lys Pro Arg Ile Pro Leu Asp
   305 310 315 320
   Val Gly His Arg Gly Ala Gly Asn Ser Thr Thr Thr Ala Gln Leu Ala
   325 330 335
   Lys Val Gln Glu Asn Thr Ile Ala Ser Leu Arg Asn Ala Ala Ser His
   340 345 350
   Gly Ala Ala Phe Val Glu Phe Asp Val His Leu Ser Lys Asp Phe Val
   355 360 365
   Pro Val Val Tyr His Asp Leu Thr Cys Cys Leu Thr Met Lys Lys Lys
   370 375 380
   Phe Asp Ala Asp Pro Val Glu Leu Phe Glu Ile Pro Val Lys Glu Leu
   385 390 395 400
   Thr Phe Asp Gln Leu Gln Leu Leu Lys Leu Thr His Val Thr Ala Leu
   405 410 415
   Lys Ser Lys Asp Arg Lys Glu Ser Val Val Gln Glu Glu Asn Ser Phe
   420 425 430
   Ser Glu Asn Gln Pro Phe Pro Ser Leu Lys Met Val Leu Glu Ser Leu
   435 440 445
   Pro Glu Asp Val Gly Phe Asn Ile Glu Ile Lys Trp Ile Cys Gln Gln
   450 455 460
   Arg Asp Gly Met Trp Asp Gly Asn Leu Ser Thr Tyr Phe Asp Met Asn
   465 470 475 480
   Leu Phe Leu Asp Ile Ile Leu Lys Thr Val Leu Glu Asn Ser Gly Lys
   485 490 495
   Arg Arg Ile Val Phe Ser Ser Phe Asp Ala Asp Ile Cys Thr Met Val
               500                 505                 510
   Arg Gln Lys Gln Asn Lys Tyr Pro Ile Leu Phe Leu Thr Gln Gly Lys
           515                 520                 525
   Ser Glu Ile Tyr Pro Glu Leu Met Asp Leu Arg Ser Arg Thr Thr Pro
       530                 535                 540
   Ile Ala Met Ser Phe Ala Gln Phe Glu Asn Leu Leu Gly Ile Asn Val
   545                 550                 555                 560
   His Thr Glu Asp Leu Leu Arg Asn Pro Ser Tyr Ile Gln Glu Ala Lys
                   565                 570                 575
   Ala Lys Gly Leu Val Ile Phe Cys Trp Gly Asp Asp Thr Asn Asp Pro
               580                 585                 590
   Glu Asn Arg Arg Lys Leu Lys Glu Leu Gly Val Asn Gly Leu Ile Tyr
           595                 600                 605
   Asp Arg Ile Tyr Asp Trp Met Pro Glu Gln Pro Asn Ile Phe Gln Val
       610                 615                 620
   Glu Gln Leu Glu Arg Leu Lys Gln Glu Leu Pro Glu Leu Lys Ser Cys
   625                 630                 635                 640
   Leu Cys Pro Thr Val Ser Arg Phe Val Pro Ser Ser Leu Cys Gly Glu
                   645                 650                 655
   Ser Asp Ile His Val Asp Ala Asn Gly Ile Asp Asn Val Glu Asn Ala
               660                 665                 670
<210> 9
   <211> 25
   <212> DNA
   <213> künstliche Sequenz
<220>
   <223> Oligonukleotid
<400> 9
   attaaccctc actaaatgct gggga 25
<210> 10
   <211> 26
   <212> DNA
   <213> künstliche Sequenz
<220>
   <223> oligonukleotid
<400> 10
   attaaccctc actaaatcgg tcatag 26
<210> 11
   <211> 25
   <212> DNA
   <213> künstliche Sequenz
<220>
   <223> Oligonukleotid
<400> 11
   attaaccctc actaaatgct ggtgg 25
<210> 12
   <211> 25
   <212> DNA
   <213> künstliche Sequenz
<220>
   <223> Oligonukleotid
<400> 12
   attaaccctc actaaatgct ggtag 25
<210> 13
   <211> 26
   <212> DNA
   <213> künstliche Sequenz
<220>
   <223> Oligonukleotid
<400> 13
   attaaccctc actaaagatc tgactg 26
<210> 14
   <211> 25
   <212> DNA
   <213> künstliche Sequenz
<220>
   <223> Oligonukleotid
<400> 14
   attaaccctc actaaatgct gggtg 25
<210> 15
   <211> 25
   <212> DNA
   <213> künstliche Sequenz
<220>
   <223> Oligonukleotid
<400> 15
   attaaccctc actaaatgct gtatg 25
<210> 16
   <211> 25
   <212> DNA
   <213> künstliche Sequenz
<220>
   <223> Oligonukleotid
<400> 16
   attaaccctc actaaatgga gctgg 25
<210> 17
   <211> 25
   <212> DNA
   <213> künstliche Sequenz
<220>
   <223> Oligonukleotid
<400> 17
   attaaccctc actaaatgtg gcagg 25
<210> 18
   <211> 25
   <212> DNA
   <213> künstliche Sequenz
<220>
   <223> Oligonukleotid
<400> 18
   attaaccctc actaaagcac cgtcc 25
<210> 19
   <211> 30
   <212> DNA
   <213> künstliche Sequenz
<220>
   <223> Oligonukleotid
<400> 19
   cattatgctg agtgatatct ttttttttaa 30
<210> 20
   <211> 30
   <212> DNA
   <213> künstliche Sequenz
<220>
   <223> Oligonukleotid
<400> 20
   cattatgctg agtgatatct ttttttttac 30
<210> 21
   <211> 30
   <212> DNA
   <213> künstliche Sequenz
<220>
   <223> Oligonukleotid
<400> 21
   cattatgctg agtgatatct ttttttttag 30
<210> 22
   <211> 30
   <212> DNA
   <213> künstliche Sequenz
<220>
   <223> Oligonukleotid
<400> 22
   cattatgctg agtgatatct ttttttttca 30
<210> 23
   <211> 30
   <212> DNA
   <213> künstliche Sequenz
<220>
   <223> Oligonukleotid
<400> 23
   cattatgctg agtgatatct ttttttttcc 30
<210> 24
   <211> 30
   <212> DNA
   <213> künstliche Sequenz
<220>
   <223> Oligonukleotid
<400> 24
   cattatgctg agtgatatct ttttttttcg 30
<210> 25
   <211> 30
   <212> DNA
   <213> künstliche Sequenz
<220>
   <223> Oligonukleotid
<400> 25
   cattatgctg agtgatatct ttttttttga 30
<210> 26
   <211> 30
   <212> DNA
   <213> künstliche Sequenz
<220>
   <223> oligonukleotid
<400> 26
   cattatgctg agtgatatct ttttttttgc 30
<210> 27
   <211> 30
   <212> DNA
   <213> künstliche Sequenz
<220>
   <223> oligonukleotid
<400> 27
   cattatgctg agtgatatct ttttttttgg 30
<210> 28
   <211> 22
   <212> DNA
   <213> künstliche Sequenz
<220>
   <223> Oligonukleotid
<400> 28
   tttcaaaatg ctgcagggta at 22
<210> 29
   <211> 22
   <212> DNA
   <213> künstliche Sequenz
<220>
   <223> Oligonukleotid
<400> 29
   acccacaaag caacagtgtg ta 22
<210> 30
   <211> 24
   <212> DNA
   <213> künstliche Sequenz
<220>
   <223> Oligonukleotid
<400> 30
   cacaatctgc ttctaatcca agaa 24
<210> 31
   <211> 22
   <212> DNA
   <213> künstliche Sequenz
<220>
   <223> Oligonukleotid
<400> 31
   tgctttgtgg gtttgttttg ta 22